# EUROPEAN PATENT APPLICATION

(11) **EP 4 431 592 A1**
(43) Date of publication of application: **18.09.2024**
(21) Application number: 22892519.4
(22) Date of filing: 19.10.2022
(51) Int. Cl.: C12M 3/00, C12N 5/07

(54) **CULTURE VESSEL AND CULTURE METHOD**

(30) Priority: 09.11.2021 JP 2021182633
(71) Applicant: Mitsui Chemicals, Inc., Tokyo 104-0028 (JP)
(72) Inventor: ESASHIKA, Katsuhiro, Sodegaura-shi, Chiba 299-0265 (JP); MIYASAKO, Hiroshi, Mobara-shi, Chiba 297-0017 (JP)
(74) Representative: J A Kemp LLP
(86) International application number: PCT/JP2022/038875
(87) International publication number: WO 2023/085019

(57) **Abstract**

An aspect of the present invention relates to a culture vessel or a culture method. The culture vessel is for culturing a cell, tissue or organ, wherein the culture vessel includes a bottom surface component and a lateral wall component, the bottom surface component and the lateral wall component are bonded to each other to form at least one culture space, at least a part of the bottom surface component includes at least an oxygen-permeable layer (I-b) and a gas barrier layer (II-b), the gas barrier layer (II-b) is stacked under the oxygen-permeable layer (I-b) so as to be detachable from the oxygen-permeable layer (I-b), the gas barrier layer (II-b) satisfies the following requirement (5), and the oxygen-permeable layer (I-b) satisfies the following requirement (6):
(5) an oxygen permeability P(II-b) at a temperature of 23°C and a humidity of 0% is 3,000 cm³/(m²·24h·atm) or less
(6) an oxygen permeability P(I-b) at a temperature of 23°C and a humidity of 0% is equal to or more than 6.0 times the P(II-b).

## Description

### Technical Field

An aspect of the present invention relates to a culture vessel and a culture method.

### Background Art

Cells, tissues or organs (hereinafter, also referred to as cells or the like) can be cultured only under conditions suitable for growth, and therefore are required to be placed in a culture vessel such as a dish, a plate or a flask together with a culture medium containing appropriate nutrients, with the culture vessel being allowed to stand in an incubator capable of maintaining the temperature, the humidity and the gas concentration at predetermined levels. Further, for efficiently performing the culture, oxygen must be sufficiently and properly supplied.

In general, culture of cells or the like under hypoxic conditions is considered undesirable because cell death is induced. Thus, culture vessels using a resin material having a high oxygen permeability have been developed for promoting supply of oxygen to cells or the like. For example, Non Patent Literature 1 reports that when liver cells having a high oxygen consumption rate were cultured with polydimethylsiloxane (PDMS) used for a bottom surface of a culture vessel, an oxygen-deficient state found in a commercially available polystyrene plate was eliminated, and a phenomenon of a high level of self-organization of liver cells was observed. Moreover, Patent Literature 1 discloses a material for culturing cells, tissues or organs, which contains a 4-methyl-1-pentene polymer and has an excellent oxygen supply property.

Surprisingly, however, for a method for culturing liver cells in which liver cells are cultured on an oxygen-permeable film, it has been revealed that since culture is performed in an environment at a low oxygen concentration, a drug transport function can be efficiently formed, and liver tissues capable of maintaining the drug transport function for a long period can be easily produced (Patent Literature 2) .

### Citation List

### Patent Literature

[Patent Literature 1] WO2020/256079
[Patent Literature 2] JP 2014-223061

### Non Patent Literature

[Non Patent Literature 1] Yasuyuki SAKAI, "Enhanced oxygen supply in hepatocyte culture", [online], The Japanese Society for the Research of Hepatic Cells, [searched on August 26, 2021], Internet <http://hepato.umin.jp/kouryu/kouryu28.html>

### Summary of Invention

### Technical Problem

The present inventors have thought that a reduced amount of oxygen supplied in an early stage of culture of cells or the like may lead to better cell attachment. In addition, the present inventors have thought that by sufficiently supplying oxygen from the middle of the culture, normal functions of cells are maintained. Accordingly, an aspect of the present invention provides a culture vessel or a culture method which allows cells or the like to easily attach to the culture vessel and enables maintenance of normal functions of cells or the like.

### Solution to Problem

The present inventors have conducted diligent research in order to solve the problems described above. As a result, the present inventors have found that the problems described above can be solved by having the following configuration, leading to completion of the present invention.

The aspect of the present invention relates to, for example, the following [1] to [7].
[1] A culture vessel for culturing a cell, tissue or organ in a culture medium, wherein at least a part of a culture surface of the culture vessel includes a laminated region of an oxygen-permeable layer (I-a) satisfying the following requirements (1) and (2) and a gas barrier layer (II-a) satisfying the following requirements (3) and (4), the gas barrier layer (II-a) is provided on a lower surface of the oxygen-permeable layer (I-a), and the gas barrier layer (II-a) is detachable from the oxygen-permeable layer (I-a) during culture:
   (1) an oxygen permeability P(I-a) at a temperature of 23°C and a humidity of 0% is 20,000 to 60,000 cm³/(m²·24h·atm)
   (2) a total light transmittance measured according to JIS K 7361-1 is 70% or more
   (3) an oxygen permeability P(II-a) at a temperature of 23°C and a humidity of 0% is 3,000 cm³/(m²·24h·atm) or less
   (4) the total light transmittance measured according to JIS K 7361-1 is 70% or more.
[2] The culture vessel according to [1], wherein a water contact angle of the oxygen-permeable layer (I-a) is 30 to 120°.
[3] The culture vessel according to [1] or [2], wherein the oxygen-permeable layer (I-a) contains a 4-methyl-1-pentene polymer (X).
[4] The culture vessel according to [3], wherein the 4-methyl-1-pentene polymer (X) is at least one polymer selected from a 4-methyl-1-pentene homopolymer (x1) and a copolymer (x2) of 4-methyl-1-pentene and at least one olefin selected from ethylene and an α-olefin having 3 to 20 carbon atoms (except for 4-methyl-1-pentene).
[5] The culture vessel according to any one of [1] to [4], wherein the culture surface is coated with a natural polymeric material, a synthetic polymeric material or an inorganic material.
[6] The culture vessel according to any one of [1] to [5], wherein the gas barrier layer (II-a) contains polyethylene terephthalate (PET).
[7] A method for culturing a cell, tissue or organ, comprising a step (A-a) of culturing a cell, tissue or organ using the culture vessel according to any one of [1] to [6], a step (B-a) of detaching the gas barrier layer (II-a) from the oxygen-permeable layer (I-a), and a step (C-a) of further culturing the cell, tissue or organ using the culture vessel obtained in the step (B-a).

The aspect of the present invention also relates to, for example, the following [i] to [xiii].
[i] A culture vessel for culturing a cell, tissue or organ, wherein the culture vessel includes a bottom surface component and a lateral wall component, the bottom surface component and the lateral wall component are bonded to each other to form at least one culture space, at least a part of the bottom surface component includes at least an oxygen-permeable layer (I-b) and a gas barrier layer (II-b), the gas barrier layer (II-b) is stacked under the oxygen-permeable layer (I-b) so as to be detachable from the oxygen-permeable layer (I-b), the gas barrier layer (II-b) satisfies the following requirement (5), and the oxygen-permeable layer (I-b) satisfies the following requirement (6):
   (5) an oxygen permeability P(II-b) at a temperature of 23°C and a humidity of 0% is 3,000 cm³/(m²·24h·atm) or less; and
   (6) an oxygen permeability P(I-b) at a temperature of 23°C and a humidity of 0% is equal to or more than 6.0 times the P(II-b).
[ii] The culture vessel according to [i], wherein the gas barrier layer (II-b) contains at least one selected from the group consisting of polyethylene terephthalate (PET) and polyolefin, and satisfies the following requirement (7) or (8):
   (7) containing a mixture of at least one selected from the group consisting of polyethylene terephthalate (PET) and polyolefin and a pressure sensitive adhesive
   (8) including a layer containing at least one selected from the group consisting of polyethylene terephthalate (PET) and polyolefin, and a layer containing a pressure sensitive adhesive.
[iii] The culture vessel according to [i] or [ii], wherein a peeling strength of the gas barrier layer (II-b) against the oxygen-permeable layer (I-b) is 0.01 to 0.20 N/25 mm as measured by the following method:
   [a test is conducted in which the gas barrier layer (II-b) having a width of 25 mm is attached to the oxygen-permeable layer (I-b), and the gas barrier layer (II-b) is peeled from the oxygen-permeable layer (I-b) at a rate of 10 mm/min at a peeling angle of 180° using a peeling tester].
[iv] The culture vessel according to any one of [i] to [iii], wherein the oxygen-permeable layer (I-b) contains a 4-methyl-1-pentene polymer (X).
[v] The culture vessel according to [iv], wherein the 4-methyl-1-pentene polymer (X) is at least one polymer selected from a 4-methyl-1-pentene homopolymer (x1) and a copolymer (x2) of 4-methyl-1-pentene and at least one olefin selected from ethylene and an α-olefin having 3 to 20 carbon atoms (except for 4-methyl-1-pentene).
[vi] The culture vessel according to any one of [i] to [v], wherein the P(I-b) is 20,000 to 60,000 cm³/(m²·24h·atm).
[vii] The culture vessel according to any one of [i] to [vi], wherein a total light transmittance of a region of the bottom surface component which includes the oxygen-permeable layer (I-b) and the gas barrier layer (II-b) is 70% or more as measured according to JIS K 7361-1.
[viii] The culture vessel according to any one of [i] to [vii], wherein a total light transmittance of the oxygen-permeable layer (I-b) is 70% or more as measured according to JIS K 7361-1.
[ix] The culture vessel according to any one of [i] to [viii], wherein a total light transmittance of the gas barrier layer (II-b) is 70% or more as measured according to JIS K 7361-1.
[x] The culture vessel according to any one of [i] to [ix], wherein a water contact angle of the oxygen-permeable layer (I-b) is 30 to 120 °.
[xi] The culture vessel according to any one of [i] to [x], wherein the culture surface of the bottom surface component is coated with a natural polymeric material, a synthetic polymeric material or an inorganic material.
[xii] A method for culturing a cell, tissue or organ, comprising a step (A-b) of culturing a cell, tissue or organ using the culture vessel according to any one of [i] to [xi], a step (B-b) of detaching the gas barrier layer (II-b) from the oxygen-permeable layer (I-b), and a step (C-b) of further culturing the cell, tissue or organ using the culture vessel obtained in the step (B-b).
[xiii] A method for producing the culture vessel according to any one of [i] to [xii], the method comprising a step of attaching the bottom surface component to the lateral wall component.

### Advantageous Effects of Invention

According to an aspect of the present invention, it is possible to provide a culture vessel and a culture method which allow cells or the like to easily attach to the culture vessel and enable maintenance of normal functions of cells or the like.

### Brief Description of Drawings

[Figure 1] Figure 1 shows conceptual views of a cross-section of a culture vessel, where the left view of Figure 1 shows a state in which a culture surface includes a gas barrier layer (II), and the right view of Figure 1 shows a state in which the gas barrier layer (II) is detached from an oxygen-permeable layer (I).
[Figure 2] Figure 2 is a picture of an observed form of frozen human liver cells in Example 1 after culturing for 24 hours.
[Figure 3] Figure 3 is a picture of an observed form of frozen human liver cells in Comparative Example 2 after culturing for 24 hours.

### Description of Embodiments

The present invention will be specifically described below.

The description "A to B" regarding a numerical value range represents A or more and B or less unless otherwise noted. For example, the description "1 to 5%" means 1% or more and 5% or less.

An aspect of the present invention is a culture vessel for culturing a cell, tissue or organ in a culture medium, wherein at least a part of a culture surface of the culture vessel includes a laminated region of
an oxygen-permeable layer (I-a) satisfying the following requirements (1) and (2), and
a gas barrier layer (II-a) satisfying the following requirements (3) and (4),
the gas barrier layer (II-a) is provided on a lower surface of the oxygen-permeable layer (I-a), and
the gas barrier layer (II-a) is detachable from the oxygen-permeable layer (I-a) during culture. The culture vessel is referred to as a culture vessel (α).
   (1) An oxygen permeability P(I-a) at a temperature of 23°C and a humidity of 0% is 20,000 to 60,000 cm³/(m²·24h·atm).
   (2) A total light transmittance measured according to JIS K 7361-1 is 70% or more.
   (3) An oxygen permeability P(II-a) at a temperature of 23°C and a humidity of 0% is 3,000 cm³/(m²·24h·atm) or less.
   (4) The total light transmittance measured according to JIS K 7361-1 is 70% or more.

Another aspect of the present invention is
a culture vessel for culturing a cell, tissue or organ, wherein the culture vessel includes a bottom surface component and a lateral wall component,
the bottom surface component and the lateral wall component are bonded to each other to form at least one culture space,
at least a part of the bottom surface component includes at least an oxygen-permeable layer (I-b) and a gas barrier layer (II-b),
the gas barrier layer (II-b) is stacked under the oxygen-permeable layer (I-b) so as to be detachable from the oxygen-permeable layer (I-b),
the gas barrier layer (II-b) satisfies the following requirement (5), and
the oxygen-permeable layer (I-b) satisfies the following requirement (6). The culture vessel is referred to as a culture vessel (β).

(5) An oxygen permeability P(II-b) at a temperature of 23°C and a humidity of 0% is 3,000 cm³/(m²·24h·atm) or less.
(6) An oxygen permeability P(I-b) at a temperature of 23°C and a humidity of 0% is equal to or more than 6.0 times the P(II-b) .

### <Cell, tissue or organ>

In the present specification, a cell, tissue or organ is also referred to simply as "cells or the like". The origin of cells or the like is not limited, and may be any living organism such as an animal, a plant, an insect, a fungus, a protist or a bacterium, but is preferably an animal or a plant, more preferably an animal, and particularly preferably a mammal. Since a culture vessel according to an aspect of the present invention is excellent in ability to attachcells or the like, the cells or the like are preferably adherent cells. The cells or the like are preferably cells because they are suitable for culturing on a culture surface of the culture vessel. In an aspect of the present invention, the cells or the like are preferably aerobic cells or the like, and more preferably cells or the like that do not include anaerobic cells or the like.

A tissue in an aspect of the present invention means similar cells aggregated to play the same role. The tissue is not limited, and examples thereof include epithelial tissues, connective tissues, muscle tissues, and neural tissues. The tissue is preferably a liver lobule, a cardiac muscle tissue, a neural tissue or a skeletal muscle tissue, and preferably a liver lobule because it has a high oxygen demanding property.

An organ in an aspect of the present invention means the tissues aggregated to perform cooperative work with a purpose. The organ is not limited, and is, for example, a lung, a heart, a liver, a kidney, a spleen, a pancreas, a gallbladder, an esophagus, a stomach, a skin, or a brain. The organ is preferably a skin, a kidney, a liver, a heart or a brain, and more preferably a liver because it has a high oxygen demanding property.

Cells in an aspect of the present invention are not limited, but are preferably animal cells.

The cells may be non-adherent cells or adherentcells, but are preferably adherentcells.

The cells may be cells that are two-dimensionally cultured or cells that are three-dimensionally cultured, and the cells include spheroid obtained by culturing cells. The cells may be primary cells in culture or a subcultured cell line, but are preferably primary cells in culture. Frozen or re-frozen cells may be used.

As the animal cells, for example, normal cells, cancer cells and fusion cells such as hybridomas may be used, and the cells may be those subjected to artificial treatment such as gene introduction. As the animal cells, for example, undifferentiated pluripotent stem cells, pluripotent stem cells in the process of differentiation, differentiated cells derived from pluripotent stem cells, pluripotent stem cells, somatic stem cells, and differentiated cells derived from animal tissues may be used.

The origin of the animal cells may be any animal, but is preferably a mammal. In particular, cells of a human, a bovine, a dog, a cat, a pig, a miniature pig, a rabbit, a hamster, a rat or mouse are preferable, and cells of a human, a rat, a mouse or a bovine are more preferable.

Since, for example, skins, kidneys, livers, brains, neural tissues, cardiac muscle tissues, skeletal muscle tissues, cancer stem cells and cancer cells have a high oxygen demanding property, and cells forming them also have a high oxygen demanding property, cells in an aspect of the present invention are preferably cells forming skins, kidneys, livers, brains, neural tissues, cardiac muscle tissues or skeletal muscle tissues, or cancer cells, more preferably liver cells, kidney cells, cardiac muscle cells or neural cells, and still more preferably liver cells.

One of these types of cells may be used singly, or two or more thereof may be used in combination.

In addition, the cells may include or exclude stem cells or precursor cells. The cells may exclude or include epithelial stem cells or precursor cells.

Liver cells in an aspect of the present invention may be any cells as long as they are cells in the liver including liver parenchymal cells (hepatocytes), and specific examples thereof include vascular endothelial cells, vascular smooth muscle cells, adipose cells, hemocyte cells, liver mononuclear cells, liver macrophages (including Kupffer cells), liver astrocytic cells, and intrahepatic bile duct epithelial cells. The liver cells are, for example, a population of cells including liver parenchymal cells at 20% or more, 30% or more, 40% or more or 50% or more.

The liver cells may be primary cells in culture or a subcultured cell line, but it is preferable to use primary cells in culture as liver cells. Examples of the type subcultured cell line is not limited, and examples thereof include SSP-25, RBE, HepG2, TGBC50TKB, HuH-6, HuH-7, ETK-1, Het-1A, PLC/PRF/5, Hep3B, SK-HEP-1, C3A, THLE-2, THLE-3, HepG2/2.2.1, SNU-398, SNU-449, SNU-182, SNU-475, SNU-387, SNU-423, FL62891, and DMS153.

The liver cells may be a population of cells including cell species other than liver cells, and are, for example, a population of cells including liver cells at 20% or more, 30% or more, 40% or more or 50% or more.

The function of cells or the like includes functions corresponding to the types of cells or the like in addition to basic functions such as cellular proliferation, repair and metabolism and exchange of information between cells. The functions of the types of cells or the like are, for example, adjustment of carbohydrates, fat synthesis, protein metabolism, alcohol metabolism, ammonia metabolism, cholesterol metabolism, drug metabolism and bile secretion in the case of liver cells. These functions can be measured by known methods, and the measured values may be used as values indicating the functions of cells or the like. The function of liver cells is preferably evaluated using a metabolic activity value, and more preferably evaluated using a metabolic activity value from CYP1A2.

It can be said that normal functions of cells or the like are maintained when the value indicating the function of cells or the like 48 hours after the cells or the like are seeded is preferably 55% or more, more preferably 60% or more, and still more preferably 65% or more, of the measured value 24 hours after the cells or the like are seeded.

### <Culture>

In an aspect of the present invention, the term "culture" is used in a broad sense including not only proliferation and maintenance of cells or the like but also processes such as seeding, subculture, induction of differentiation and induction of self-organization of cells or the like. A culture medium or the like for use in culture is not limited, and a culture medium corresponding to the characteristics of cells or the like may be selected.

Culture of cells or the like may be two-dimensional culture (including a case where cells are autonomously layered), or three-dimensional culture. In a culture vessel according to an aspect of the present invention, the amount of oxygen supplied can be changed during culture to perform culturing in a suitable oxygen environment in conformity to a state of cells or the like, so that not only in two-dimensional culture but also in three-dimensional culture where cells are sterically stacked, cells proliferate and differentiate, and a phenomenon of a high level of self-organization of cells is likely to occur.

The three-dimensional culture means sterically culturing cells by intention, and may be a scaffold type in which cells are cultured in a scaffold material, or a scaffold-free type in which cells are cultured in a floating state as a mass (spheroid), but the scaffold type is preferable. In the case of the scaffold type, Matrigel^{™}, collagen gel, laminin, alginate hydrogel and vitrigel are preferable as scaffold materials because cells can be efficiently cultured.

The culture medium or the like for use in culture is not limited, but it is preferable to culture cells in the presence of serum (for example, fetal bovine serum) for efficiently culturing cells.

The cell seeding density in culturing performed using a culture vessel according to an aspect of the present invention is not limited as long as cells can proliferate and differentiate, which is preferably 0.1 × 10⁵ cells/cm² to 10.0 × 10⁵ cells/cm², more preferably 0.3 × 10⁵ cells/cm² to 5.0 × 10⁵ cells/cm², and still more preferably 0.5 × 10⁵ cells/cm² to 3.0 × 10⁵ cells/cm².

When the cell seeding density is in the above-described range, cells more favorably attach to the vessel and normal functions of cells are more easily maintained as compared to cases where the cell seeding density is outside the above-described range.

### <Culture vessel>

With regard to the culture vessel (α), the culture vessel means all vessels that are used for culturing a cell, tissue or organ in a culture medium. Culturing in a culture medium means that at least a part of cells or the like is immersed in a culture medium and cultured, and all cells or the like need not be immersed in the culture medium.

With regard to the culture vessel (β), the culture vessel means all vessels that are used for culturing a cell, tissue or organ. The culturing may be performed in a culture medium.

As the culture vessel, various known culture vessels may be used, and the shape and the size thereof are not limited. Examples of the culture vessel include dishes, flasks, plates, bottles, bags, and tubes. The culture vessel is typically used inside an apparatus such as an incubator, a mass culture apparatus or a perfusion culture.

The culture vessel is preferably a dish, a flask or a plate, and more preferably a plate having at least one well.

The culture vessel is preferably a culture vessel having at least one well, more preferably a plate having at least one well, and still more preferably a plate having a plurality of wells such as 6 wells, 12 wells, 24 wells, 48 wells, 96 wells, 384 wells and 1536 wells. Generally, in a culture vessel having a concave shape such as a well on a bottom surface, it is necessary to thicken the bottom surface for stabilizing the complicated shape of the bottom surface, and it is difficult to sufficiently supply oxygen to cells or the like. When the bottom surface includes a laminated region of the oxygen-permeable layer (I-a) and the gas barrier layer (II-a) as in the culture vessel (α), the shape is stable and the amount of oxygen supplied to cells or the like is easily controlled in a plate having one well or even a plate having a plurality of wells such as 6 wells, 12 wells, 24 wells, 48 wells, 96 wells, 384 wells and 1536 wells. When at least a part of a bottom surface component includes at least the oxygen-permeable layer (I-b) and the gas barrier layer (II-b) and the gas barrier layer (II-b) is stacked under the oxygen-permeable layer (I-b) as in the culture vessel (β), similarly, the shape is stable and the amount of oxygen supplied to cells or the like is easily controlled even in the case of a plate having a plurality of wells.

The culture vessel (β) includes a bottom surface component and a lateral wall component, and the bottom surface component and the lateral wall component are bonded to each other to form at least one culture space. In this case, the bottom surface component is bonded to the lateral wall component to form the bottom surface of the culture vessel. The culture space is a space confined to culture cells or the like, in other words, a space in which cells or the like cultured in the space can freely move.

The bottom surface component and the lateral wall component are bonded to each other so that cells or the like or a culture medium in the culture space does not leak. The bonding may be any of mechanical bonding, material bonding and adhesive bonding, but adhesive bonding is preferable because it is easy to combine a bottom surface component and a lateral wall component which include desired materials.

In the case where the culture vessel (β) is a culture vessel having at least one well, the culture vessel may include, for example, a bottom surface component and a well lateral wall component, with the bottom surface component and the well lateral wall component being bonded to each other to form at least one well. In this case, the bottom surface component is bonded to the well lateral wall component to form the bottom surface of the well.

The culture vessel is preferably a culture vessel in which the bottom surface includes a culture surface for holding or storing a culture medium.

Here, the culture surface means, among portions forming the culture vessel, a portion that is in contact with the culture medium and/or cells or the like or a portion that is expected to be in contact with the culture medium and/or cells or the like in culture of cells or the like. For example, in the case where the culture vessel is a dish, a flask or a plate, typically, a bottom surface portion includes a culture surface, and the culture medium and/or cells or the like come into contact with the upper side of the culture surface, that is, the inside of the culture vessel.

The shape of the bottom surface of the culture vessel is not limited, and examples thereof include a flat bottom (F bottom), a round bottom (U bottom), a conical bottom (V bottom), and a flat bottom with a curved edge. In the case of processing into, for example, a round bottom (U bottom), a flat bottom (F bottom), a conical bottom (V bottom), or a flat bottom with a curved edge, the processing may be performed at a time by common injection molding or press molding, and it is also possible to produce the culture vessel by producing a film or a sheet, and performing secondary processing by, for example, vacuum molding or pressure molding. The shape of the bottom surface is selected according to a purpose of culture, but in two-dimensional culture of cells or the like, a flat bottom (F bottom) is typically desirable, and in three-dimensional culturing, a round bottom (U bottom) or a conical bottom (V bottom) is typically desirable.

In the culture vessel, at least a culture surface thereof, preferably a culture surface thereof on a side where a contact with the culture medium and/or cells or the like is made, may be coated with a natural polymeric material, a synthetic polymeric material or an inorganic material. The coating can be performed by a known method.

The coated culture vessel has higher ability to attach and proliferate cells or the like. This may be because the natural polymeric material, the synthetic polymeric material or the inorganic material coated to the culture surface serves as a scaffold for cells or the like. Therefore, when cells or the like are attached and cultured, in the culture vessel, a culture surface is preferably coated with a natural polymeric material, a synthetic polymeric material or an inorganic material, and a culture surface on a side where a contact with the culture medium and/or cells or the like is made is more preferably coated with a natural polymeric material, a synthetic polymeric material or an inorganic material.

The natural polymeric material, synthetic polymeric material or inorganic material is not limited. Examples of the natural polymeric material include collagen, gelatin, alginic acid, hyaluronic acid, glycosaminoglycans such as chondroitin sulfate, fibronectin, laminin, fibrinogen, osteopontin, tenascin, vitronectin, thrombospondin, agarose, elastin, keratin, chitosan, fibrin, fibroin, and saccharides. Examples of the synthetic polymeric material include polyglycolic acid, polylactic acid, polyethylene glycol, polycaprolactone, synthetic peptides, synthetic proteins, polyhydroxyethyl methacrylate, and polyethyleneimine. Examples of the inorganic material include tricalcium β-phosphate, and calcium carbonate.

In addition, examples of the natural polymeric material, synthetic polymeric material or inorganic material include vitrigel obtained by vitrifying hydrogel such as a conventional extracellular matrix component, and then performing rehydration. Mention is also made of, for example, collagen vitrigel formed of high-density collagen fiber networks and produced from collagen that is one of extracellular matrix components.

From the viewpoint of, for example, improving the ability to attach or proliferate cells or the like and maintaining functions of cells or the like for a longer period, coating with a protein such as collagen, gelatin, laminin or polylysine, or peptide is preferable, coating treatment with laminin, collagen or polylysine is more preferable, and coating treatment with collagen is further more preferable. One of these coating treatments may be performed singly, or two or more thereof may be performed in combination.

In the culture vessel, fine processing may be performed on a culture surface, preferably a culture surface on a side where a contact with the culture medium and/or cells or the like is made, for forming spheroid and improving the scaffolding function of cells or the like. A method for performing the fine processing can be appropriately selected from, for example, cutting and processing, an optical lithography method, an electron beam direct drawing method, a particle beam processing method, a scanning probe processing method, self-organization of fine particles, a nanoimprint method using a master formed by any of the foregoing methods, molding methods represented by a casting method or an injection molding method, and a plating method. The pattern of the fine processing is not limited, but the height from a groove portion to a peak portion is preferably 20 nm to 500 um. The thickness of the thinnest portion of the culture vessel can be reduced to 20 um as compared to cases where fine processing is not performed on the surface.

A culture vessel on which fine processing has been performed may be used as a microchannel device (also referred to as a microchannel chip). The microchannel device is a generic term of devices for applying fine processing to a culture surface of a culture vessel to produce microchannels and reaction vessels, which are to be applied to biological studies and chemical engineering. Examples thereof include apparatuses called microTAS (Micro Total Analysis Systems) and Lab on a Chip, which are being applied as next-generation culture apparatuses.

In the culture vessel, surface modification treatment is preferably performed on at least a culture surface thereof, preferably a culture surface thereof on a side where a contact with the culture medium and/or cells or the like is made.

The method for use in the surface modification treatment is not limited, and examples thereof include hydrophilization treatments such as corona treatment, plasma treatment, ozone treatment and ultraviolet treatment; hydrophobization treatments such as esterification, silanization and fluorination, surface graft polymerization, chemical vapor deposition, etching, or addition of a specific functional group such as a hydroxyl group, an amino group, a sulfone group, a thiol group or a carboxyl group, treatment with a specific functional group, such as silane coupling, titanium coupling and zirconium coupling, surface roughening by such as oxidants, and physical treatments such as rubbing and sand blasting. One of these surface modification treatments may be performed singly, or two or more thereof may be performed in combination.

It is preferable that in the culture vessel, hydrophilization treatment, more preferably corona treatment or plasma treatment, be performed on at least a culture surface thereof, preferably a culture surface thereof on a side where a contact with the culture medium and/or cells or the like is made. By hydrophilization treatment is performed on the surface of the culture vessel, the wettability of the surface of the culture vessel is enhanced, and attachment between the culture vessel and cells or the like is improved, so that cells or the like can uniformly proliferate on the surface of the culture vessel. By performing hydrophilization treatment on the surface of the culture vessel, the culture surface of the culture vessel is easily coated with a natural polymeric material, a synthetic polymeric material or an inorganic material. In particular, a natural polymeric material, a synthetic polymeric material or an inorganic material is easily deposited uniformly on the culture surface of the culture vessel, and brought into close contact with the culture surface, and after the deposition treatment, the natural polymeric material, synthetic polymeric material or inorganic material does not peel during washing with physiological saline and in a cell culture environment, and can be used in cell culture while maintaining a stable initial state.

In the case where plasma treatment is performed, as a gas to be entrained, nitrogen, hydrogen, helium, oxygen or argon, for example, is used, and at least one gas selected from nitrogen, helium and argon is preferably selected.

Disinfection or sterilization treatment may be applied to the culture vessel for preventing contamination. The method for disinfection or sterilization treatment is not limited, and examples thereof include physical disinfection methods such as a free-flowing steam method, a boiling method, an intermittent method and an ultraviolet method; chemical disinfection methods using a gas such as ozone or a disinfectant such as ethanol; heat sterilization methods such as a high-pressure steam method and a dry heat method; irradiation sterilization methods such as a gamma ray method, an electron beam method and a high-frequency method; and gas sterilization methods such as an ethylene oxide gas method and a hydrogen gas plasma method. Among them, an ethanol disinfection method, a high-pressure steam sterilization method, a gamma ray sterilization method, an electron beam sterilization or an ethylene oxide gas sterilization method are preferable because they involve easy operations and enable sufficient sterilization. One of these disinfection or sterilization treatments may be performed singly, or two or more thereof may be performed in combination.

### <Oxygen-permeable layer (I)>

An oxygen-permeable layer (I-a) and an oxygen-permeable layer (I-b) described below are collectively referred to as an oxygen-permeable layer (I).

### <Oxygen-permeable layer (I-a)>

The oxygen-permeable layer (I-a) is a layer that satisfies the following requirements (1) and (2).
(1) An oxygen permeability P(I-a) at a temperature of 23°C and a humidity of 0% is 20,000 to 60,000 cm³/(m²·24h·atm).
(2) A total light transmittance measured according to JIS K 7361-1 is 70% or more.

### [Requirement (1)]

The oxygen permeability P(I-a) can be specifically measured by the following method.

A measurement sample is produced from the oxygen-permeable layer (I-a), an oxygen permeation coefficient [cm³·mm/(m²·24h·atm)] at a temperature of 23°C and a humidity of 0% is measured by a differential pressure type gas permeation rate measurement method. Equipment for use in the measurement is not limited as long as it is based on a differential pressure type gas permeation rate measurement method, and examples thereof include a differential pressure type gas permeation rate measurement apparatus MT-C3 manufactured by Toyo Seiki Seisaku-sho, Ltd. The measurement sample is produced by cutting out a 90 × 90 mm test piece with a thickness of 50 um from the oxygen-permeable layer (I-a), and the diameter of a measurement portion is preferably 70 mm (the permeation area is 38.46 cm²). Since the oxygen permeability is high, it is preferable to set the actual permeation area to 5.0 cm² by applying an aluminum mask to the sample in advance. The measurement sample may be a measurement sample on which fine processing and surface modification treatment have or have not been performed, but a measurement sample on which any treatment has not been performed is preferable. A value obtained by dividing an oxygen permeation coefficient by the thickness of the film (µm) is taken as the oxygen permeability [cm³/(m²·24h·atm)].

The oxygen permeability P(I-a) of the oxygen-permeable layer (I-a) is preferably 20,000 to 50,000 cm³/(m²·24h·atm), more preferably 25,000 to 45,000 cm³/(m²·24h·atm), and still more preferably 30,000 to 40,000 cm³/(m²·24h·atm). When the oxygen permeability P(I-a) is in the above-described range, it can be said that the oxygen-permeable layer (I-a) is excellent in oxygen permeability.

If the oxygen permeability P(I-a) of the oxygen-permeable layer (I-a) is smaller than the lower limit, the concentration of oxygen in the culture medium decreases, so that cells or the like do not sufficiently proliferate and differentiate. On the other hand, if the oxygen permeability P(I-a) is larger than the upper limit, the concentration of oxygen in the culture medium becomes excessively high, so that cell functions are deteriorated by oxygen stress. When the oxygen permeability P(I-a) is in a range between the upper and lower limits, cells or the like can efficiently proliferate and differentiate depending on a culture period, and maintain cell functions.

The oxygen permeability P(I-a) of the oxygen-permeable layer (I-a) can be adjusted by adjusting, for example, the thickness of the oxygen-permeable layer (I-a) or materials for forming the oxygen-permeable layer (I-a).

### [Requirement (2)]

The total light transmittance can be specifically measured by the following method.

With a test piece produced from the oxygen-permeable layer (I-a), a total amount of transmitted light is measured according to JIS K 7361-1 using a hazemeter HM-150 (D65 light source) manufactured by Murakami Color Research Laboratory Co., Ltd., and the total light transmittance is determined from the following equation. Total light transmittance (%) = 100 × (total amount of transmitted light) / (amount of incident light)

The total light transmittance of the oxygen-permeable layer (I-a) is preferably 90.0% or more, and more preferably 92.0% or more. The upper limit of the total light transmittance is not defined, but is typically 99.9%. When the total light transmittance is in the above-described range, it can be said that the oxygen-permeable layer (I-a) is excellent in transparency.

If the total light transmittance of the oxygen-permeable layer (I-a) is below the above-described range, the oxygen-permeable layer (I-a) has low transparency, so that it is difficult to observe cells or the like in the culture vessel. When the total light transmittance is in a range between the upper and lower limits, the oxygen-permeable layer (I-a) is excellent in transparency, so that cells or the like are easily observed either with the naked eye or under a microscope.

The total light transmittance of the oxygen-permeable layer (I-a) can be adjusted by adjusting, for example, the thickness of the oxygen-permeable layer (I-a) or materials for forming the oxygen-permeable layer (I-a).

### <Oxygen-permeable layer (I-b)>

The oxygen-permeable layer (I-b) is a layer that satisfies the following requirement (6).

### [Requirement (6)]

An oxygen permeability P(I-b) at a temperature of 23°C and a humidity of 0% is equal to or more than 6.0 times P(II-b) described later.

### [Oxygen permeability P(I-b)]

The oxygen permeability P(I-a) can be measured in the same manner as in the case of the oxygen permeability P(I-a) and in a suitable mode.

The oxygen permeability P(I-b) of the oxygen-permeable layer (I-b) is preferably 20,000 to 60,000 cm³/(m²·24h·atm), more preferably 20,000 to 50,000 cm³/(m²·24h·atm), still more preferably 25,000 to 45,000 cm³/(m²·24h·atm), and particularly preferably 30,000 to 40,000 cm³/(m²·24h·atm). When the oxygen permeability P(I-b) is in the above-described range, it can be said that the oxygen-permeable layer (I-b) is excellent in oxygen permeability.

If the oxygen permeability P(I-b) of the oxygen-permeable layer (I-b) is less than 6.0 times the oxygen permeability P (II-b) of a gas barrier layer (II-b) described later, the concentration of oxygen in the culture medium decreases, so that cells or the like do not sufficiently proliferate and differentiate. On the other hand, when the oxygen permeability P(I-b) is 60,000 cm³/(m²·24h·atm) or less, the concentration of oxygen in the culture medium can be prevented from becoming excessively high, so that deterioration of functions of cells by oxygen stress is easily suppressed. When the oxygen permeability P(I-b) is in a range between the upper and lower limits, cells or the like can efficiently proliferate and differentiate depending on a culture period, and maintain cell functions.

The oxygen permeability P(I-b) of the oxygen-permeable layer (I-b) can be adjusted by adjusting, for example, the thickness of the oxygen-permeable layer (I-b) or materials for forming the oxygen-permeable layer (I-b).

### [Total light transmittance of oxygen-permeable layer (I-b)]

The total light transmittance of the oxygen-permeable layer (I-b) can be measured in the same manner as in the case of the oxygen-permeable layer (I-a).

The total light transmittance of the oxygen-permeable layer (I-b) is preferably 70% or more, more preferably 90.0% or more, and still more preferably 92.0% or more. The upper limit of the total light transmittance is not defined, but is typically 99.9%. When the total light transmittance is in the above-described range, it can be said that the oxygen-permeable layer (I-b) is excellent in transparency.

If the total light transmittance of the oxygen-permeable layer (I-b) is below the above-described range, the oxygen-permeable layer (I-b) has low transparency, so that it is difficult to observe cells or the like in the culture vessel. When the total light transmittance is in a range between the upper and lower limits, the oxygen-permeable layer (I-b) is excellent in transparency, so that cells or the like are easily observed either with the naked eye or under a microscope.

The total light transmittance of the oxygen-permeable layer (I-b) can be adjusted by adjusting, for example, the thickness of the oxygen-permeable layer (I-b) or materials for forming the oxygen-permeable layer (I-b).

Typically, the culture medium and/or cells or the like are in contact with the upper surface of the oxygen-permeable layer (I).

The water contact angle of the oxygen-permeable layer (I) is preferably 30° or more, more preferably 35° or more, still more preferably 40° or more, and particularly preferably 45° or more, and preferably 120° or less, more preferably 110° or less, still more preferably 100° or less, particularly preferably 84° or less, and most preferably 70° or less.

When the water contact angle of the oxygen-permeable layer (I) is adjusted to be in the above-described range, for example, cells or the like easily attach to the oxygen-permeable layer (I), and cells or the like easily proliferate uniformly on the oxygen-permeable layer (I). In addition, a natural polymeric material, a synthetic polymeric material or an inorganic material is easily coated uniformly onto the oxygen-permeable layer (I), and brought into close contact with the oxygen-permeable layer (I), and after the coating, the natural polymeric material, synthetic polymeric material or inorganic material does not peel during washing with physiological saline and in a cell culture environment, and can be used in cell culture while maintaining a stable initial state.

The method for measuring the water contact angle is not limited, and may be a known method, but is preferably a static drop method. The water contact angle can be measured by, for example, a method in which according to Japanese Industrial Standard JIS-R3257 (Testing method wettability of glass substrate), a water droplet with a volume of 4 µL or less which can be considered to have a spherical shape under constant temperature and humidity conditions of 25 ± 5°C and 50 ± 10% is dropped to a surface of a measurement sample, and by a static drop method, the angle of a contact interface between the measurement sample and the water droplet within 1 minute immediately after the water droplet comes into contact with the surface of the measurement sample is measured.

The water contact angle of the oxygen-permeable layer (I) can be adjusted by adjusting, for example, materials forming the oxygen-permeable layer (I) and conditions for surface modification treatment of the oxygen-permeable layer (I) .

The material for forming the oxygen-permeable layer (I-a) is not limited as long as it can form the oxygen-permeable layer (I-a) that satisfies the requirements (1) and (2).

The material for forming the oxygen-permeable layer (I-b) is not limited as long as it can form the oxygen-permeable layer (I-b) that satisfies the requirement (6).

One of the materials for forming the oxygen-permeable layer (I) may be used singly, or two or more may be used in combination.

For the material for forming the oxygen-permeable layer (I), an appropriate material can be selected from the viewpoint of, for example, moldability, transparency, shape stability, lightweight properties, low-drug-sorption properties, autofluorescence and radiation resistance. When from such a viewpoint, a polyolefin-based resin or a fluorine-based resin, more preferably a polyolefin-based resin, is selected, a culture vessel according to an aspect of the present invention efficiently cultures cells or the like, facilitates observation of the form of cells or the like, and is easily used in drug development screening applications and diagnostic applications.

Examples of the fluorine-based resin include polytetrafluoroethylene (PTFE), polytrifluorochloroethylene, polyvinyl fluoride, polyvinylidene fluoride, dichlorodifluoroethylene, polychlorotrifluoroethylene, fluorinated ethylene-propylene copolymers, perfluoroalkyl vinyl ether polymers, perfluoroalkyl vinyl ester polymers, ethylene-tetrafluoroethylene copolymers, and polydimethylsiloxane (PDMS).

Examples of the polyolefin-based resin that can be used for the oxygen-permeable layer (I) include homopolymers or copolymers of an α-olefin such as ethylene, 1-butene, 1-hexene, 4-methyl-1-pentene or 1-octene; high-pressure low-density polyethylene; linear low-density polyethylene (LLDPE); high-density polyethylene; polypropylene (homopolymer of propylene); copolymers of propylene and an α-olefin having 2 or more and 10 or less carbon atoms; ethylene/vinyl acetate copolymers (EVAs); and ionomer resins; and fluorine-containing cyclic olefin copolymers.

Polyolefin-based resins are preferable because they are excellent in balance of, for example, moldability, transparency, shape stability, lightweight properties, low-drug-sorption properties and autofluorescence, and among them, a 4-methyl-1-pentene polymer (X) described later is preferable. That is, the oxygen-permeable layer (I) preferably contains a 4-methyl-1-pentene polymer (X).

### [4-Methyl-1-pentene polymer (X)]

In an aspect of the present invention, 4-methyl-1-pentene homopolymers and copolymers of 4-methyl-1-pentene and other monomers are collectively referred to as a "4-methyl-1-pentene polymer (X)".

The copolymer of 4-methyl-1-pentene and other monomers which is an example of the 4-methyl-1-pentene polymer (X) may be any of a random copolymer, an alternate copolymer, a block copolymer and a graft copolymer. As the copolymer of 4-methyl-1-pentene and other monomers, copolymers of 4-methyl-1-pentene and at least one olefin selected from ethylene and an α-olefin having 3 to 20 carbon atoms (except for 4-methyl-1-pentene) are preferable because they have high strength, are unlikely to break when used as the oxygen-permeable layer (I), and hardly warp.

The 4-methyl-1-pentene polymer (X) is preferably at least one polymer selected from a 4-methyl-1-pentene homopolymer, and a copolymer of 4-methyl-1-pentene and at least one olefin selected from ethylene and an α-olefin having 3 to 20 carbon atoms (except for 4-methyl-1-pentene), and more preferably a copolymer of 4-methyl-1-pentene and at least one olefin selected from ethylene and an α-olefin having 3 to 20 carbon atoms (except for 4-methyl-1-pentene).

Examples of the olefin include ethylene, propylene, 1-butene, 1-hexene, 1-heptene, 1-octene, 1-decene, 1-tetradecene, 1-hexadecene, 1-heptadecene, 1-octadecene, and 1-eicosene. The olefin can be appropriately selected according to physical properties necessary for the oxygen-permeable layer (I). For example, from the viewpoint of moderate oxygen permeability and excellent rigidity, the olefin is preferably an α-olefin having 8 to 18 carbon atoms, and more preferably at least one selected from 1-octene, 1-decene, 1-dodecene, 1-tetradecene, 1-hexadecene, 1-heptadecene and 1-octadecene. When the number of carbons in the olefin is in the above-described range, the processability of the polymer is further improved, and deterioration in appearance of the oxygen-permeable layer (I) due to cracking and breakage of end portions hardly occurs. The occurrence ratio of defective oxygen-permeable layers (I) decreases.

One or more of the olefins may be used. From the viewpoint of the strength of the material, the number of carbon atoms is preferably 2 or more, and more preferably 10 or more. In the case where two or more different α-olefins are combined, it is particularly preferable to combine at least one selected from 1-tetradecene and 1-hexadecene and at least one selected from 1-heptadecene and 1-octadecene.

The content of constitutional units derived from 4-methyl-1-pentene in the 4-methyl-1-pentene polymer (X) is preferably 60 to 100 mol%, more preferably 80 to 99.5 mol%, and still more preferably 85 to 98 mol%.

In the case where the 4-methyl-1-pentene polymer (X) is a copolymer of 4-methyl-1-pentene and at least one olefin selected from ethylene and an α-olefin having 3 to 20 carbon atoms (except for 4-methyl-1-pentene), the content of constitutional units derived from at least one olefin selected from ethylene and an α-olefin having 3 to 20 carbon atoms (except for 4-methyl-1-pentene) in the copolymer is preferably 0 to 40 mol%, more preferably 0.5 to 20 mol%, and still more preferably 2 to 15 mol%. Note that the content of these constitutional units is based on the amount of all repeating constitutional units in the 4-methyl-1-pentene polymer (X), which is defined as 100 mol%. When the content of the constitutional units is within the above-described range, a culture surface that is excellent in processability and homogeneous is obtained, and warpage hardly occurs because the film has well-balanced toughness and strength.

The 4-methyl-1-pentene polymer (X) may have constitutional units other than the constitutional units derived from 4-methyl-1-pentene and the constitutional units derived from ethylene and an α-olefin having 3 to 20 carbon atoms (hereinafter, also referred to as "other constitutional units") as long as the effects of the present invention are not impaired. The content of other constitutional units is, for example, 0 to 10.0 mol%. In the case where the 4-methyl-1-pentene polymer (X) has other constitutional units, there may be one or more types of constitutional units.

Examples of the monomer from which other constitutional units are derived include cyclic olefins, aromatic vinyl compounds, conjugated dienes, nonconjugated polyenes, functional vinyl compounds, hydroxyl group-containing olefins, and halogenated olefins. As the cyclic olefins, aromatic vinyl compounds, conjugated dienes, nonconjugated polyenes, functional vinyl compounds, hydroxyl group-containing olefins, and halogenated olefins, for example, compounds described in JP2013-169685, paragraphs [0035]-[0041] may be used.

One of the 4-methyl-1-pentene polymers (X) may be used singly, or two or more may be used in combination.

As the 4-methyl-1-pentene polymer (X), a commercially available product may be used. Specific examples thereof include TPX MX001, MX002, MX004, MX002O, MX021, MX321, RT18, RT31 or DX845 (all of which are trademarks). Even a product from another manufacturer can be preferably used as long as it is the 4-methyl-1-pentene polymer (X) satisfying the above-described requirements. One of these commercially available products may be used singly, or two or more thereof may be used in combination.

The 4-methyl-1-pentene polymer (X) typically has a melting point of 200°C to 240°C and high heat resistance. Since the 4-methyl-1-pentene polymer (X) does not undergo hydrolysis and is excellent in water resistance, boiling water resistance and steam resistance, high-pressure steam sterilization treatment is applicable to the oxygen-permeable layer (I) containing the 4-methyl-1-pentene polymer (X). Since the 4-methyl-1-pentene polymer (X) is characterized in that the light transmittance is high (typically 90% or more) and autofluorescence is not emitted, a culture vessel formed from the oxygen-permeable layer (I) containing the 4-methyl-1-pentene polymer (X) ensures easy observation of cultured cells. Further, since the 4-methyl-1-pentene polymer (X) exhibits excellent resistance to almost all chemicals, and hardly absorb a drug, the 4-methyl-1-pentene polymer (X) does not interfere with the effects of the drug for maintaining cells or the like, and are suitably used in drug development screening applications and diagnostic application. Since the 4-methyl-1-pentene polymer (X) has high strength, a culture vessel formed from the oxygen-permeable layer (I) containing the 4-methyl-1-pentene polymer (X) ensures is unlikely to break, and hardly warps. The 4-methyl-1-pentene polymer (X) can be heat-sealed, and is easy not only to heat-seal to itself but also to heat-bond to another material. In addition, the 4-methyl-1-pentene polymer (X) can be thermally molded, and therefore is easy to mold into a culture vessel with an arbitrary shape, and easy to mold using, for example, an imprinting method or an insert method.

The weight-average molecular weight (Mw) of the 4-methyl-1-pentene polymer (X) measured by gel permeation chromatography (GPC) using standard polystyrene as a reference substance is preferably 10,000 to 2,000,000, more preferably 20,000 to 1,000,000, and still more preferably 30,000 to 500,000. Here, the sample concentration in GPC measurement may be, for example, 1.0 to 5.0 mg/mL. The molecular weight distribution (Mw/Mn) of the 4-methyl-1-pentene polymer (X) is preferably 1.0 to 30, more preferably 1.1 to 25, still more preferably 1.1 to 20. The solvent for use in GPC is preferably ortho-dichlorobenzene. Examples of the measurement conditions include, but are not limited to, conditions shown in Examples described later.

When the weight-average molecular weight (Mw) is equal to or less than the upper limit, occurrence of defects such as gel is easily suppressed and a film with a uniform surface is easily formed in the case where the film is produced by melt molding in a later-described method for molding the 4-methyl-1-pentene polymer (X). In production by a solution-casting method, solubility in a solvent is further improved, defects such as gel in the film are easily suppressed, and a film with a uniform surface is easily formed.

When the weight-average molecular weight (Mw) is equal to or more than the lower limit, the culture vessel tends to have sufficient strength. Further, when the molecular weight distribution is within the above-described range, stickiness of the surface of the produced culture vessel is easily suppressed, the culture vessel tends to have sufficient toughness, and for example, occurrence of bending during molding and cracking during cutting is easily suppressed.

The weight average molecular weight (Mw) and the molecular weight distribution (Mw/Mn) of the 4-methyl-1-pentene polymers (X), in the case where two or more 4-methyl-1-pentene polymers (X) are used, may be such that Mw and Mw/Mn of each of the two or more 4-methyl-1-pentene polymers (X) are in the above-described ranges.

The oxygen permeation coefficient of the 4-methyl-1-pentene (X) is preferably 1,000 to 3,000 cm³·mm/(m²·24h·atm), and more preferably 1,000 to 2,500 cm³·mm/(m²·24h·atm). When the oxygen permeation coefficient is in the above-described range, excellent oxygen permeability is exhibited, so that cells or the like maintain a good form, and are likely to efficiently proliferate depending on a culture period.

Since the 4-methyl-1-pentene polymer (X) has excellent characteristics as described above, a culture vessel in which at least a culture surface has the oxygen-permeable layer (I) containing the 4-methyl-1-pentene polymer (X) does not have a harmful effect on culture, has good shape stability, transparency, moldability and oxygen permeability, can be sterilized, and is extremely excellent as a culture vessel for culturing cells or the like.

### [Method for producing 4-methyl-1-pentene polymer (X)]

The method for producing the 4-methyl-1-pentene polymer (X) may be any method as long as 4-methyl-1-pentene, an olefin and other monomers can be polymerized. A chain transfer agent, for example, hydrogen may coexist for controlling the molecular weight and the molecular weight distribution. Equipment for use in the production is not limited. The polymerization method may be a known method, or may be a gas phase method, a slurry method, a solution method or a bulk method. A slurry method and a solution method are preferable. The polymerization method may be a single-stage polymerization method, or a two-stage or multi-stage polymerization method, where a plurality of polymers having different molecular weights are blended in a polymerization system. In any of single-stage and multi-stage polymerization methods, in the case where hydrogen is used as the chain transfer agent, it may be collectively introduced, or separately introduced, for example, introduced in the early stage, the middle stage and the end stage of polymerization. The polymerization may be performed at room temperature, and heat may be applied if necessary, but from the viewpoint of efficiency of the polymerization, the polymerization is performed preferably at 20°C to 80°C, and more preferably at 40°C to 60°C. The catalyst for use in the production is not limited, but from the viewpoint of the efficiency of the polymerization, it is preferable to use, for example, a solid titanium catalyst component (I) described in WO2006/054613 or a catalyst for polymerization of olefin (metallocene catalyst) containing a transition metal compound (A) which is described in WO2014/050817.

Note that in the case where the oxygen-permeable layer (I) containing the 4-methyl-1-pentene polymer (X) comprises a composition containing the 4-methyl-1-pentene polymer (X), the amount of the 4-methyl-1-pentene polymer (X) is preferably 90% by mass or more and less than 100% by mass, more preferably 95% by mass and less than 100% by mass, and particularly preferably 99% by mass or more and less than 100 mass%, in 100% by mass of the composition. The inclusion of a large amount of components other than the 4-methyl-1-pentene polymer (X) causes not only a decrease in oxygen permeability, but also a decrease in transparency or a decrease in strength.

Examples of the component other than the 4-methyl-1-pentene polymer (X) include additives such as heat resistant stabilizers, light resistant stabilizers, processing aids, plasticizers, antioxidants, lubricants, defoaming agents, antiblocking agents, colorants, modifiers, antibacterial agents, antimold agents, and anti-fogging agents.

The oxygen-permeable layer (I) may have a single-layer structure with only one layer, or a multi-layer structure with two or more layers.

In the case where the oxygen-permeable layer (I) has a multi-layer structure, the constitutional materials and the thicknesses of the layers may be the same or different.

The thickness of the oxygen-permeable layer (I-a) is not limited as long as the oxygen permeability at a temperature of 23°C and a humidity of 0% is 20,000 to 60,000 cm³/(m²·24h·atm), which is preferably 20 to 500 µm, more preferably 25 to 500 µm, and still more preferably 30 to 200 um.

The thickness of the oxygen-permeable layer (I-b) is not limited as long as the requirement (6) is satisfied, which is preferably 20 to 500 µm, more preferably 25 to 500 µm, and still more preferably 30 to 200 µm.

Note that the thickness, in the case where the oxygen-permeable layer (I) has a multi-layer structure, refers to a total thickness of the layers.

When the thickness of the oxygen-permeable layer (I) is within the above-described range, excellent strength is achieved. In particular, even if the culture vessel has a small number of wells and a large hole diameter, warpage is unlikely to occur. In addition, breakage is unlikely to occur in application of, for example, corona treatment.

### <Gas barrier layer (II)>

A gas barrier layer (II-a) and a gas barrier layer (II-b) described below are collectively referred to as a gas barrier layer (II).

### <Gas barrier layer (II-a)>

The gas barrier layer (II-a) is a layer that satisfies the following requirements (3) and (4).
(3) An oxygen permeability P(II-a) at a temperature of 23°C and a humidity of 0% is 3,000 cm³/(m²·24h·atm) or less.
(4) The total light transmittance measured according to JIS K 7361-1 is 70% or more.

### [Requirement (3)]

The oxygen permeability P(II-a) can be measured in the same manner as in the case of the oxygen-permeable layer (I-a) .

The oxygen permeability P(II-a) of the gas barrier layer (II-a) is preferably more than 1 cm³/(m²·24h·atm) and 3,000 cm³/ (m²·24h·atm) or less, more preferably more than 1 cm³/(m²·24h·atm) and 1,000 cm³/(m²·24h·atm) or less, and still more preferably more than 1 cm³/ (m²·24h·atm) and 500 cm³/ (m²·24h·atm) or less, and particularly preferably more than 1 cm³/(m²·24h·atm) and 300 cm³/(m²·24h·atm) or less.

When the oxygen permeability P(II-a) is in the above-described range, it can be said that the gas barrier layer (II-a) is excellent in gas barrier properties.

If the oxygen permeability P(II-a) of the gas barrier layer (II-a) is excessively large, the concentration of oxygen in the culture medium immediately after cells or the like are seeded becomes excessively high, and the attachment of cells or the like is deteriorated. If the oxygen permeability P(II-a) of the gas barrier layer (II-a) is excessively low, the attachment of cells or the like is deteriorated, and in subsequent culture, cell functions tend not to be maintained. When the oxygen permeability P(II-a) is in a range between the upper and lower limits, cells or the like favorably attach to the culture vessel.

The oxygen permeability P(II-a) of the gas barrier layer (II-a) can be adjusted by adjusting, for example, the thickness of the gas barrier layer (II-a) or materials for forming the gas barrier layer (II-a).

### [Requirement (4)]

The total light transmittance can be measured in the same manner as in the case of the oxygen-permeable layer (I-a) .

The total light transmittance of the gas barrier layer (II-a) is preferably 85.0% or more, and more preferably 90.0% or more. The upper limit of the total light transmittance is not defined, but is typically 99.9%. When the total light transmittance is in the above-described range, it can be said that the gas barrier layer (II-a) is excellent in transparency.

If the total light transmittance is excessively small, the gas barrier layer (II-a) has low transparency, so that it is difficult to observe cells or the like in the culture vessel. When the total light transmittance is in a range between the upper and lower limits, the gas barrier layer (II) is excellent in transparency, so that cells or the like are easily observed either with the naked eye or under a microscope.

The total light transmittance of the gas barrier layer (II-a) can be adjusted by adjusting, for example, the thickness of the gas barrier layer (II-a) or materials for forming the gas barrier layer (II-a).

### <Gas barrier layer (II-b)>

The gas barrier layer (II-b) is a layer that satisfies the following requirement (5).

### [Requirement (5)]

An oxygen permeability P(II-b) at a temperature of 23°C and a humidity of 0% is 3,000 cm³/(m²·24h·atm) or less.

### [Oxygen permeability P(II-b)]

The oxygen permeability P(II-b) can be measured in the same manner as in the case of the oxygen permeability P(I-a) and in a suitable mode.

The suitable range and adjustment method for the oxygen permeability P(II-b) of the gas barrier layer (II-b) are the same as in the case of the gas barrier layer (II-a).

### [Total light transmittance of gas barrier layer (II-b)]

The total light transmittance of the gas barrier layer (II-b) can be measured in the same manner as in the case of the oxygen-permeable layer (I-a).

The total light transmittance of the gas barrier layer (II-b) is preferably 70% or more, more preferably 85.0% or more, and still more preferably 90.0% or more. The upper limit of the total light transmittance is not defined, but is typically 99.9%. When the total light transmittance is in the above-described range, it can be said that the gas barrier layer (II-b) is excellent in transparency.

If the total light transmittance is excessively small, the gas barrier layer (II-b) has low transparency, so that it is difficult to observe cells or the like in the culture vessel. When the total light transmittance is in a range between the upper and lower limits, the gas barrier layer (II-b) is excellent in transparency, so that cells or the like are easily observed either with the naked eye or under a microscope.

The total light transmittance of the gas barrier layer (II-b) can be adjusted by adjusting, for example, the thickness of the gas barrier layer (II-b) or materials for forming the gas barrier layer (II-b).

The material for forming the gas barrier layer (II-a) is not limited as long as it can form the gas barrier layer (II-a) that satisfies the requirements (3) and (4).

Examples of the material for forming the gas barrier layer (II-a) include polyethylene terephthalate (PET), nylon, ethylene-vinyl alcohol copolymers (EVOHs), polyglycolic acid, aromatic polyamide, and polyvinylidene chloride (PVDC), and among them, an appropriate material can be selected from the viewpoint of, for example, moldability, transparency, shape stability, lightweight properties, low-drug-sorption properties, autofluorescence and radiation resistance. When from such a viewpoint, polyethylene terephthalate is selected, a culture vessel according to an aspect of the present invention efficiently cultures cells or the like, facilitates observation of the form of cells or the like, and is easily used in drug development screening applications and diagnostic applications. That is, the gas barrier layer (II-a) preferably contains polyethylene terephthalate (PET).

One of the materials for forming the gas barrier layer (II-a) may be used singly, or two or more may be used in combination.

The material for forming the gas barrier layer (II-b) is not limited as long as it can form the gas barrier layer (II-b) that satisfies the requirement (5).

Examples of the material for forming the gas barrier layer (II-b) include polyethylene terephthalate (PET), nylon, ethylene-vinyl alcohol copolymers (EVOHs), polyglycolic acid, aromatic polyamide, polyvinylidene chloride (PVDC), and polyolefins, and among them, an appropriate material can be selected from the viewpoint of, for example, moldability, transparency, shape stability, lightweight properties, low-drug-sorption properties, autofluorescence and radiation resistance. When from such a viewpoint, polyethylene terephthalate or polyolefin is selected, a culture vessel according to an aspect of the present invention efficiently cultures cells or the like, facilitates observation of the form of cells or the like, and is easily used in drug development screening applications and diagnostic applications. That is, the gas barrier layer (II-b) preferably contains at least one selected from the group consisting of polyethylene terephthalate (PET) and polyolefin, and more preferably contains polyethylene terephthalate (PET).

One of the materials for forming the gas barrier layer (II-b) may be used singly, or two or more may be used in combination.

The gas barrier layer (II-a) may be a non-stretched film, or a stretched film such as a uniaxially stretched film or a biaxially stretched film. More specific examples of such a film include single-layer or multi-layer stretched films such as biaxially stretched polyethylene terephthalate films and biaxially stretched nylon films, films comprising, for example, an ethylene-vinyl alcohol copolymer (EVOH), polyglycolic acid, aromatic polyamide or polyvinylidene chloride (PVDC), and various polyvinylidene-coated films.

The gas barrier layer (II-b) may be a non-stretched film, or a stretched film such as a uniaxially stretched film or a biaxially stretched film. More specific examples of such a film include single-layer or multi-layer stretched films such as biaxially stretched polyethylene terephthalate films and biaxially stretched nylon films, films comprising, for example, an ethylene-vinyl alcohol copolymer (EVOH), polyglycolic acid, aromatic polyamide or polyvinylidene chloride (PVDC), various polyvinylidene-coated films, and polyolefin films including, for example, polyethylene (PE) or polypropylene (PP).

### [Polyethylene terephthalate]

The polyethylene terephthalate (PET) is a polymer obtained by polycondensation of at least one selected from terephthalic acid and an ester-forming derivative thereof and at least one selected from ethylene glycol and an ester-forming derivative thereof. Examples of the ester-forming derivative of terephthalic acid include alkyl esters of terephthalic acid such as dimethyl terephthalate. Examples of the ester-forming derivative of ethylene glycol include fatty acid esters such as ethylene glycol fatty acid esters.

As long as effects obtained in the present invention are not impaired, the polyethylene terephthalate may be obtained by copolymerization of at least one selected from terephthalic acid and an ester-forming derivative thereof with at least one selected from another dicarboxylic acid and an ester-forming derivative thereof, or copolymerization of at least one selected from ethylene glycol and an ester-forming derivative thereof with at least one selected from another diol and an ester-forming derivative thereof. Examples of the dicarboxylic acid and ester-forming derivative thereof for use as a copolymerization component include isophthalic acid, adipic acid, oxalic acid, sebacic acid, decanedicarboxylic acid, naphthalenedicarboxylic acid, and alkyl esters thereof. Examples of the diol and ester-forming derivative for use as a copolymerization component include ethylene glycol, propylene glycol, neopentyl glycol, 1,5-pentanediol, 1,6-hexanediol, decamethylene glycol, cyclohexane dimethanol, cyclohexane diol, long-chain glycols such as polyethylene glycol, poly-1,3-propylene glycol and polytetramethylene glycol having a molecular weight of 400 to 6,000, and fatty acid esters thereof. One of these copolymerization components may be used singly, or two or more thereof may be used in combination. The amount of these copolymerization components is preferably 20% by mass or less of the amount of a raw materials for forming polyethylene terephthalate.

The type of polyethylene terephthalate is not limited as long as effects obtained in the present invention are not impaired, which is preferably crystalline polyethylene terephthalate (C-PET) from the viewpoint of mechanical strength and formability.

The method for producing polyethylene terephthalate is not limited, and may be a known method.

### [Polyolefin]

Examples of the polyolefin that can be used as a material for forming the gas barrier layer (II-b) include homopolymers or copolymers of an α-olefins such as ethylene, 1-butene, 1-hexene, 4-methyl-1-pentene or 1-octene; highpressure low-density polyethylene; linear low-density polyethylene (LLDPE); high-density polyethylene; polypropylene (homopolymer of propylene); copolymers of propylene and an α-olefin having 2 or more and 10 or less carbon atoms; ethylene/vinyl acetate copolymers (EVAs); and ionomer resins; and fluorine-containing cyclic olefin copolymers. Among them, homopolymers or copolymers of propylene are preferable. That is, the gas barrier layer (II-b) preferably contains a homopolymer or a copolymer of propylene, more preferably contains polypropylene (a homopolymer of propylene).

The constitutional unit other than constitutional units derived from propylene in the copolymer of propylene is not limited, and may be at least one olefin selected from ethylene and an α-olefin having 4 to 20 carbon atoms. Examples of the olefin include ethylene, 1-butene, 1-hexene, 1-heptene, 1-octene, 1-decene, 1-tetradecene, 1-hexadecene, 1-heptadecene, 1-octadecene, and 1-eicosene. The olefin can be appropriately selected according to physical properties necessary for the gas barrier layer (II-b).

In the case where film-shaped polyethylene terephthalate and/or polyolefin are used for the gas barrier layer (II), the film-shaped polyethylene terephthalate and/or polyolefin may or need not be stretched. The film-shaped polyethylene terephthalate and/or polyolefin are crystallographically oriented when stretched, and are therefore preferable from the viewpoint of mechanical properties. The method of stretching is preferably biaxial stretching.

Note that in the case where the gas barrier layer (II) containing polyethylene terephthalate and/or polyolefin comprises a composition containing polyethylene terephthalate and/or polyolefin, the amount of the polyethylene terephthalate and polyolefin is preferably 90% by mass or more and less than 100% by mass, more preferably 95% by mass or more and less than 100% by mass, and particularly preferably 99% by mass or more and less than 100 mass%, in 100% by mass of the composition. The inclusion of a large amount of components other than polyethylene terephthalate and polyolefin causes not only an increase in oxygen permeability, but also a decrease in transparency or a decrease in strength.

Examples of the component other than the polyethylene terephthalate and polyolefin include additives such as heat resistant stabilizers, light resistant stabilizers, processing aids, plasticizers, antioxidants, lubricants, defoaming agents, antiblocking agents, colorants, modifiers, antibacterial agents, antimold agents, anti-fogging agents, and adhesives.

The gas barrier layer (II) may have a single-layer structure with only one layer, or a multi-layer structure with two or more layers.

In the case where the gas barrier layer (II) has a multi-layer structure, the constitutional materials and the thicknesses of the layers may be the same or different.

In the case where the gas barrier layer (II) has a multi-layer structure, one of the layers may be vapor-deposited film layer formed of an inorganic oxide. This enables further improvement of the gas barrier properties of the gas barrier layer (II). Examples of the inorganic oxide include aluminum oxide (alumina), silicon oxide (silica), magnesium oxide, calcium oxide, zirconium oxide, titanium oxide, boron oxide, hafnium oxide, barium oxide, and silicon carbide oxide (carbon-containing silicon oxide).

The thickness of the gas barrier layer (II) is not limited as long as the oxygen permeability at a temperature of 23°C and a humidity of 0% is 3,000 cm³/(m²·24h·atm) or less, which is preferably 20 to 500 µm, more preferably 25 to 500 µm, and still more preferably 30 to 200 um. Note that the thickness, in the case where the gas barrier layer (II) has a multi-layer structure, refers to a total thickness of the layers.

When the thickness of the gas barrier layer (II) is within the above-described range, excellent strength is achieved. In particular, even if the culture vessel has a small number of wells and a large hole diameter, warpage is unlikely to occur. In addition, good handleability is exhibited and breakage is unlikely to occur in detachment of the gas barrier layer (II) from the oxygen-permeable layer (I) .

It is preferable that the gas barrier layer (II-b) contain at least one selected from the group consisting of polyethylene terephthalate (PET) and polyolefin, and satisfy the following requirement (7) or (8):
(7) containing a mixture of at least one selected from the group consisting of polyethylene terephthalate (PET) and polyolefin and a pressure sensitive adhesive
(8) including a layer containing at least one selected from the group consisting of polyethylene terephthalate (PET) and polyolefin, and a layer containing a pressure sensitive adhesive.

### [Requirement (7)]

The phrase "containing a mixture of at least one selected from the group consisting of polyethylene terephthalate (PET) and polyolefin and a pressure sensitive adhesive" means that one of the gas barrier layers (II-b) comprises a composition of at least one selected from the group consisting of polyethylene terephthalate (PET) and polyolefin and a pressure sensitive adhesive.

The content of at least one selected from the group consisting of polyethylene terephthalate (PET) and polyolefin in the composition is not limited.

The content of the pressure sensitive adhesive in the composition is not limited.

The mass ratio between at least one selected from the group consisting of polyethylene terephthalate (PET) and polyolefin and the pressure sensitive adhesive is not limited.

The pressure sensitive adhesive will be described later.

### [Requirement (8)]

The phrase "including a layer containing at least one selected from the group consisting of polyethylene terephthalate (PET) and polyolefin and a layer containing a pressure sensitive adhesive" means that the gas barrier layer (II-b) includes at least two layers: a layer containing at least one selected from the group consisting of polyethylene terephthalate (PET) and polyolefin and a layer containing an adhesive.

The thickness of the layer containing at least one selected from the group consisting of polyethylene terephthalate (PET) and polyolefin is not limited, but is preferably 10 to 500 µm, and more preferably 50 to 100 um.

The thickness of the layer containing a pressure sensitive adhesive is not limited, but is preferably 1 to 100 µm, and more preferably 5 to 20 µm.

The thickness ratio between the layer containing at least one selected from the group consisting of polyethylene terephthalate (PET) and polyolefin and the layer containing a pressure sensitive adhesive is not limited, but is preferably 500 : 1 to 10 : 100, and preferably 100 : 5 to 50 : 20.

The pressure sensitive adhesive will be described later.

### <laminated region of oxygen-permeable layer (I-a) and gas barrier layer (II-a)>

In the culture vessel (α), at least a part of the culture surface of the culture vessel includes a laminated region of the oxygen-permeable layer (I-a) satisfying requirements (1) and (2) and the gas barrier layer (II-a) satisfying requirements (3) and (4).

Since the culture vessel (α) includes the gas barrier layer (II-a), permeation of oxygen into the vessel is suppressed. When the gas barrier layer (II-a) is detached from the oxygen-permeable layer (I-a) to expose the oxygen-permeable layer (I-a), the culture vessel (α) allows oxygen to sufficiently permeate into the vessel.

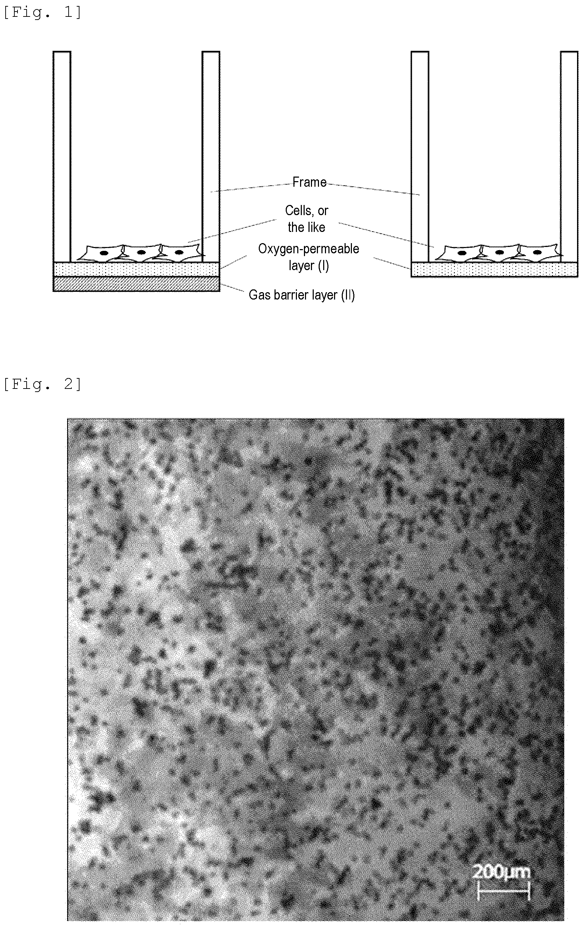

Figure 1 shows a conceptual view of a cross-section of a culture vessel according to an aspect of the present invention. The left part of Figure 1 shows a state in which the culture surface includes the gas barrier layer (II), and the right part of Figure 1 shows a state in which the gas barrier layer (II) is detached from an oxygen-permeable layer (I).

The phrase "at least a part of the culture surface includes a laminated region of the oxygen-permeable layer (I-a) and the gas barrier layer (II-a)" in the culture vessel (α) means that at least some regions of the culture surface are laminated regions of the oxygen-permeable layer (I-a) and the gas barrier layer (II-a), and all regions of the culture surface may be laminated regions of the oxygen-permeable layer (I-a) and the gas barrier layer (II-a), or only some regions of the culture surface may be laminated regions of the oxygen-permeable layer (I-a) and the gas barrier layer (II-a).

In the case where only some regions of the culture surface are laminated regions of the oxygen-permeable layer (I-a) and the gas barrier layer (II-a), it is preferable that a region of the culture surface other than the laminated region have a region comprising the oxygen-permeable layer (I-a), and it is more preferable that all of regions of the culture surface other than the laminated region be regions comprising the oxygen-permeable layer (I-a).

In the case where the culture vessel (α) is, for example, a dish, a flask or a plate, the bottom surface typically includes a culture surface, and therefore, a part or the whole of at least the bottom surface, among the bottom surface, the lateral surfaces and the upper surface of the culture vessel, includes a laminated region of the oxygen-permeable layer (I-a) and the gas barrier layer (II-a). When a part or the whole of at least the bottom surface includes a laminated region of the oxygen-permeable layer (I-a) and the gas barrier layer (II-a), oxygen supplied into a culture medium through the laminated region is easily controlled.

In the culture vessel (α), the entire culture surface preferably includes a laminated region of the oxygen-permeable layer (I-a) and the gas barrier layer (II-a). That is, in the case where the culture vessel (α) is, for example, a dish, a flask or a plate, the bottom surface includes a culture surface, and therefore, it is preferable that the whole of the bottom surface, among the bottom surface, the lateral surfaces and the upper surface of the culture vessel, include a laminated region of the oxygen-permeable layer (I-a) and the gas barrier layer (II-a).

The ratio of the laminated region of the oxygen-permeable layer (I-a) and the gas barrier layer (II-a) to the region of the culture surface is not limited, but is preferably 80% or more, more preferably 90% or more, and still more preferably 95% or more. When the ratio of the laminated region of the oxygen-permeable layer (I-a) and the gas barrier layer (II-a) to the region of the culture surface is within the above-described range, the amount of oxygen supplied to cells or the like is more easily controlled.

In the culture vessel (α), the gas barrier layer (II-a) is provided on a lower surface of the oxygen-permeable layer (I-a). That is, in the laminated region of the oxygen-permeable layer (I-a) and the gas barrier layer (II-a), the oxygen-permeable layer (I-a) and the gas barrier layer (II-a) are laminated in the mentioned order from the inner side of the culture vessel (α).

The region of the oxygen-permeable layer (I-a) and the region of the gas barrier layer (II-a) may have the same size, or one of the regions may be larger in size, but the region of the gas barrier layer (II-a) is preferably equal to or more than the region of the oxygen-permeable layer (I-a) in size for easily controlling the amount of oxygen supplied and easily detaching the gas barrier layer (II-a).

For easily controlling the amount of oxygen supplied and easily detaching the gas barrier layer (II-a), it is preferable that the region of the gas barrier layer (II-a) have a larger area over the culture surface, and it is more preferable to provide the gas barrier layer (II-a) over the entire bottom surface of the culture vessel. For example, in the case where the culture vessel is a plate having wells, it is preferable that the region of the gas barrier layer (II-a) have an area larger than the total area of the bottom surfaces of the wells, and it is preferable to provide the gas barrier layer (II-a) over the entire bottom surface of the plate.

The materials for forming components of the culture vessel (α) other than the culture surface are not limited, and may be known materials. Examples such materials include polystyrene (PS), polydimethylsiloxane (PDMS), thermosetting resins, cyclic olefin polymers, cyclic olefin copolymers, and glass.

The materials for forming components of the culture surface of the culture vessel (α) other than the laminated region of the oxygen-permeable layer (I-a) and the gas barrier layer (II-a) are not limited, and may be known materials. Examples such materials include polystyrene (PS), polydimethylsiloxane (PDMS), thermosetting resins, cyclic olefin polymers, cyclic olefin copolymers, and glass. The materials for forming components of the culture surface of the culture vessel (α) other than the laminated region of the oxygen-permeable layer (I-a) and the gas barrier layer (II-a) are preferably the same as the materials for forming the oxygen-permeable layer (I-a).

The laminated region of the oxygen-permeable layer (I-a) and the gas barrier layer (II-a) may include other layers if necessary. Examples of the other layers include an adhesive layer formed between the oxygen-permeable layer (I-a) and the gas barrier layer (II-a).

### <Oxygen-permeable layer (I-b) and gas barrier layer (II-b)>

In the culture vessel (β), at least a part the bottom surface component includes at least the oxygen-permeable layer (I-b) and the gas barrier layer (II-b), and the gas barrier layer (II-b) is provided under the oxygen-permeable layer (I-b).

Since the culture vessel (β) includes the gas barrier layer (II-b), permeation of oxygen into the vessel is suppressed. When the gas barrier layer (II-b) is detached from the oxygen-permeable layer (I-b) to expose the oxygen-permeable layer (I-b), the culture vessel (β) allows oxygen to sufficiently permeate into the vessel.

The phrase "at least a part of the bottom surface component includes at least the oxygen-permeable layer (I-b) and the gas barrier layer (II-b)" in the culture vessel (β) means that at least some regions of the bottom surface component are regions including both the oxygen-permeable layer (I-b) and the gas barrier layer (II-b), and all regions of the bottom surface component may be regions including the oxygen-permeable layer (I-b) and the gas barrier layer (II-b), or only some regions of the bottom surface component may be regions including the oxygen-permeable layer (I-b) and the gas barrier layer (II-b).

In the case where only some regions of the bottom surface component are regions including the oxygen-permeable layer (I-b) and the gas barrier layer (II-b), a region other than the region including both the layers in the bottom surface component is preferably a region comprising the oxygen-permeable layer (b), and all regions other than the region including both the layers in the bottom surface component are more preferably regions comprising the oxygen-permeable layer (I-b).

In the case where the culture vessel (β) is, for example, a dish, a flask or a plate, the bottom surface typically includes a culture surface, and therefore, at least a part of the culture surface is a region including the oxygen-permeable layer (I-b) and the gas barrier layer (II-b). When at least a part of the culture surface is a region including the oxygen-permeable layer (I-b) and the gas barrier layer (II-b), oxygen supplied into a culture medium through the region is easily controlled.

In the culture vessel (β), the entire culture surface is preferably a region including the oxygen-permeable layer (I-b) and the gas barrier layer (II-b).

The ratio of the region including the oxygen-permeable layer (I-b) and the gas barrier layer (II-b) to the region of the culture surface is not limited, but is preferably 80% or more, more preferably 90% or more, and still more preferably 95% or more. When the ratio of the region including the oxygen-permeable layer (I-b) and the gas barrier layer (II-b) to the region of the culture surface is within the above-described range, the amount of oxygen supplied to cells or the like is more easily controlled.

In the culture vessel (β), the gas barrier layer (II-b) is stacked under the oxygen-permeable layer (I-b). That is, the oxygen-permeable layer (I-b) and the gas barrier layer (II-b) are stacked in the mentioned order from the inner side of the culture vessel (β).

The gas barrier layer (II-b) may be stacked directly under the oxygen-permeable layer (I-b), or other layers (for example, a metallized layer and the adhesive layer) may be present between the oxygen-permeable layer (I-b) and the gas barrier layer (II-b). The oxygen-permeable layer (I-b), the gas barrier layer (II-b) and the other layer may be stacked in the mentioned order from the inner side of the culture vessel (β).

The region of the oxygen-permeable layer (I-b) and the region of the gas barrier layer (II-b) may have the same size, or one of the regions may be larger in size, but the region of the gas barrier layer (II-b) is preferably equal to or more than the region of the oxygen-permeable layer (I-b) in size for easily controlling the amount of oxygen supplied and easily detaching the gas barrier layer (II-b).

For easily controlling the amount of oxygen supplied and easily detaching the gas barrier layer (II-b), it is preferable that the region of the gas barrier layer (II-b) have a larger area over the culture surface, and it is more preferable to provide the gas barrier layer (II-b) over the entire bottom surface of the culture vessel. For example, in the case where the culture vessel is a plate having wells, it is preferable that the region of the gas barrier layer (II-b) have an area larger than the total area of the bottom surfaces of the wells, and it is preferable to provide the gas barrier layer (II-b) over the entire bottom surface of the plate.

The materials for forming components of the culture vessel (β) other than the bottom surface component (for example, a lateral wall component) are not limited, and may be known materials. Examples such materials include polystyrene (PS), polydimethylsiloxane (PDMS), thermosetting resins, cyclic olefin polymers, cyclic olefin copolymers, and glass.

The materials for forming regions of the bottom surface component of the culture vessel (β) other than the region including the oxygen-permeable layer (I-b) and the gas barrier layer (II-b) are not limited, and may be known materials. Examples such materials include polystyrene (PS), polydimethylsiloxane (PDMS), thermosetting resins, cyclic olefin polymers, cyclic olefin copolymers, and glass. The materials for forming regions of the bottom surface component of the culture vessel (β) other than the region including the oxygen-permeable layer (I-b) and the gas barrier layer (II-b) are preferably the same as the materials for forming the oxygen-permeable layer (I-b).

The total light transmittance of the region of the bottom surface component of the culture vessel (β) which includes the oxygen-permeable layer (I-b) and the gas barrier layer (II-b) is preferably 70.0% or more, more preferably 90.0% or more, and still more preferably 92.0% or more. The upper limit of the total light transmittance is not defined, but is typically 99.9%. When the total light transmittance is in the above-described range, it can be said that the bottom surface component is excellent in transparency.

When the total light transmittance of the region of the bottom surface component of the culture vessel (β) which includes the oxygen-permeable layer (I-b) and the gas barrier layer (II-b) is in a range between the upper and lower limits, the bottom surface component is excellent in transparency, so that cells or the like are easily observed either with the naked eye or under a microscope.

The total light transmittance of the region of the bottom surface component of the culture vessel (β) which includes the oxygen-permeable layer (I-b) and the gas barrier layer (II-b) can be adjusted by adjusting, for example, the thicknesses of the oxygen-permeable layer (I-b) and the gas barrier layer (II-b) or materials for forming the oxygen-permeable layer (I-b) and the gas barrier layer (II-b).

### <Adhesive layer>

The adhesive material for forming the adhesive layer is not limited as long as it can form an adhesive layer having desired adhesiveness, which may be an adhesive material that is commonly used for forming an adhesive layer of a device for use in, for example, cell culture and medical applications.

The adhesive material may be, for example, a pressure sensitive adhesive. Examples of the pressure sensitive adhesive include acryl-based pressure sensitive adhesives, epoxy-based pressure sensitive adhesives, and silicone-based pressure sensitive adhesives.

The adhesive material may be, for example, a heretofore known sealant resin. Examples of the sealant resin include polyolefin-based resins such as polyethylene such as low-density polyethylene (LDPE), linear low-density polyethylene (LLDPE) and high-density polyethylene (HDPE), acid-modified polyethylene, polypropylene (PP), acid-modified polypropylene, copolymers of propylene, ethylene-vinyl acetate copolymers, polypropylene-vinyl acetate copolymers, ethylene-(meth)acrylic acid ester copolymers, ethylene-(meth)acrylic acid copolymers, and ionomers which are metal-crosslinked products thereof. Among them, polyolefin-based resins are preferable from the viewpoint of low-temperature sealing property, and polyethylene is particularly preferable because it is inexpensive.

One of these adhesive materials may be used singly, or two or more thereof may be used in combination.

The thickness of the adhesive layer is only required to ensure desired adhesiveness, and can be appropriately set according to, for example, a constitutional material.

The thickness of the adhesive layer may be within the range of 0.1 µm to 200 µm, and is preferably 5 µm to 100 µm, more preferably 10 to 60 µm, and particularly preferably 15 µm to 40 µm. When the thickness of the adhesive layer is within the above-mentioned range, the adhesive layer can be excellent in processability and heat-sealing properties.

### <Method for producing culture vessel>

The method for producing the culture vessel (α) or the culture vessel (β) is not limited, and equipment for use in the production is not limited.

In the case where all components of the culture vessel (α) are formed from a material for forming the oxygen-permeable layer (I-a), the culture vessel (α) can be produced by, for example, forming a film or a sheet containing the material for forming the oxygen-permeable layer (I-a), molding the film or sheet into a desired shape if necessary, and then laminating the gas barrier layer (II-a). The culture vessel (α) can also be obtained by directly molding a material for forming the oxygen-permeable layer (I-a), by a method such as extrusion molding, solution casting molding, injection molding or blow molding, and then laminating the gas barrier layer (II-a).

In the case where some components of the culture vessel (α) or the culture vessel (β) are formed from a material for forming the oxygen-permeable layer (I), the culture vessel (α) or the culture vessel (β) can be obtained by, for example, preparing a bonded component obtained by appropriately bonding a film or a sheet containing the material for forming the oxygen-permeable layer (I) and another component to each other, and then laminating or attaching the gas barrier layer (II) to the bonded component. The bonding method is not limited, and a component containing a material for forming the oxygen-permeable layer (I) and the other component may be welded to each other, or brought into close contact with each other with an adhesive or a pressure sensitive adhesive.

The culture vessel (α) or the culture vessel (β) can also be obtained by forming a film or a sheet containing a material for forming the oxygen-permeable layer (I) and a film or a sheet containing a material for forming the gas barrier layer (II), then forming a laminated film or sheet of the two films or sheets, and appropriately bonding the laminated film or sheet and another component to each other.

In the case where the bottom surface component and the lateral wall component contains a material for forming the oxygen-permeable layer (I-b) in the culture vessel (β), the culture vessel (β) can be produced by, for example, forming a film or a sheet containing the material for forming the oxygen-permeable layer (I-b), molding the film or sheet into a desired shape if necessary, and then attaching the gas barrier layer (II-b). The culture vessel (β) can also be obtained by directly molding a material for forming the oxygen-permeable layer (I-b) into a desired shape, by a method such as extrusion molding, solution casting molding, injection molding or blow molding, and then attaching the gas barrier layer (II-b).

The method for producing the culture vessel (β) may comprise a step of attaching the bottom surface component to the lateral wall component. For example, the culture vessel (β) can be produced by attaching a film or a sheet containing a material for forming the oxygen-permeable layer (I-b) to the lateral wall component to bond the bottom surface component and the lateral wall component to each other, and then further attaching, under the film or sheet, a film or a sheet containing a material for forming the gas barrier layer (II-b). The culture vessel (β) can also be produced by attaching, in advance, a film or a sheet containing a material for forming the gas barrier layer (II-b) under a film or a sheet containing a material for forming the oxygen-permeable layer (I-b), and attaching the two attached films or sheets to the lateral wall component to bond the bottom surface component and the lateral wall component to each other.

Specifically, for example, a common inflation method or a T-die extrusion method is adopted as a method for forming the film or sheet. Typically, heat is applied during the production. In the case where a T-die extrusion method is adopted, the extrusion temperature is preferably 100°C to 400°C, and particularly preferably 200°C to 300°C. The roll temperature is preferably 45°C to 75°C, and particularly preferably 55°C to 65°C.

The film or sheet may be produced by a solution casting method in which a material is dissolved in a solvent, the solution is spread over resin or metal, and slowly dried while being leveled, thereby forming a film (forming a sheet). The solvent used is not limited, and may be a hydrocarbon solvent such as cyclohexane, hexane, decane or toluene. Two or more types of solvents may be mixed in consideration of the solubility and the drying efficiency of the material. The polymer solution is applied by a method such as table coating, spin coating, dip coating, die coating, spray coating, bar coating, roll coating or curtain flow coating, dried, and peeled to perform processing into a film or a sheet.

### <Detachable during culture>

The term "during culture" means an arbitrary time during the period of culture of cells or the like.

The term "detachable" means that attachment and detachment can be performed by normal ability of the user of the culture vessel.

The method for detachably laminating the gas barrier layer (II-a) to the oxygen-permeable layer (I-a), or the method for stacking the gas barrier layer (II-b) under the oxygen-permeable layer (I-b) so as to be detachable from the oxygen-permeable layer (I-b) is not limited, and may be a known method. Examples thereof include methods such as fixation with a magnetic force, fixation by adhesion, mechanical engagement, holding with a resilient spring component, or locking with a screw component. Among them, fixation by adhesion is preferable because the gas barrier layer (II) is stably laminated to or stacked on the oxygen-permeable layer (I), and easily attached and detached.

The gas barrier layer (II) is preferably detachable from the oxygen-permeable layer (I) by 90% or more, more preferably by 95% or more, and still more preferably by 98% or more of regions laminated to or stacked on the oxygen-permeable layer (I). When the ratio of detachable regions of the gas barrier layer (II) to regions of the gas barrier layer (II) which are laminated to or stacked on the oxygen-permeable layer (I) is within the above-described range, the amount of oxygen supplied to cells or the like is more easily controlled. Note that the gas barrier layer (II) may be detached from the oxygen-permeable layer (I) at a time, or in two or more steps, and the ratio of detachable regions of the gas barrier layer (II) to regions of the gas barrier layer (II) which are laminated to or stacked on the oxygen-permeable layer (I) is represented by a numerical value obtained by integration from the two or more detachments.

The time to detach the gas barrier layer (II) from the oxygen-permeable layer (I) may be an appropriate time determined by a type of cells or the like and culture conditions, and is not limited, but is typically a time after cells or the like are seeded, a time after it is confirmed with a microscope that cells or the like have attached to the culture vessel, or a time when cells or the like are expected to attach to the culture vessel.

After being detached from the oxygen-permeable layer (I), the gas barrier layer (II) may be attached again to the lower surface of the oxygen-permeable layer (I).

All of the regions of the gas barrier layer (II-b) may be detached from the oxygen-permeable layer (I-b), or only a part of the regions of the gas barrier layer (II-b) may be detached from the oxygen-permeable layer (I-b). That is, a part or all of the regions of the gas barrier layer (II-b) may be detachable from the oxygen-permeable layer (I-b).

As a mode in which only a part of the regions of the gas barrier layer (II-b) is detachable from the oxygen-permeable layer (I-b), for example, in the case where the culture vessel is a plate having at least one well, grid-like cuts corresponding to the wells are made in the gas barrier layer (II-b) in advance, and only the gas barrier layer (II-b) corresponding to wells in which sufficient oxygen is to be supplied to cells is detached. In the case where the culture vessel is a plate having at least one well, mention is also made of a mode in which a part (for example, 1/4, 1/3 or 1/2) of the regions of the gas barrier layer (II-b), that is, only regions corresponding to a certain number of wells among the wells of the plate (for example, 1/4, 1/3 or 1/2) are detached to expose the oxygen-permeable layer (I-b) in the certain number of wells, and a mode in which only a part (for example, 1/4, 1/3 or 1/2) of regions corresponding to the wells of the gas barrier layer (II-b) is detached to expose a part (for example, 1/4, 1/3 or 1/2) of the regions of the oxygen-permeable layer (I-b) in the wells.

### [Peeling strength]

The peeling strength of the gas barrier layer (II-b) against the oxygen-permeable layer (I-b) is preferably 0.01 to 0.20 N/25 mm, and more preferably 0.02 to 0.15 N/25 mm. When the peeling strength is equal to or more than the lower limit, unintended peeling, for example, peeling of the gas barrier layer (II-b) under its own weight from the oxygen-permeable layer (I-b) can be prevented. On the other hand, when the peeling strength is equal to or less than the upper limit, it is possible to prevent damage to cells or the like due to impacts during peeling, for example, damage to cells or the like which is caused by peeling sound-induced vibration or vibration of the hand holding the culture vessel.

The peeling strength can be specifically measured by the following method.

A 25 mm-wide test piece produced from the gas barrier layer (II-b) is attached, while a surface used for measuring the peeling strength faces downward, to the upper surface of the oxygen-permeable layer (I-b), and a 2 kg roller is made to reciprocate once. This is held in an environment at 23°C and RH 50% for 30 minutes, and the peeling strength [N/25 mm] is then measured under conditions of a peeling angle of 180° and a pulling rate of 10 mm/min in an environment at 23°C and RH 50% according to JIS Z 0237 using a peeling tester. As the peeling tester, for example, Strograph E-S manufactured by Toyo Seiki Seisaku-sho, Ltd. may be used. The test is conducted three times, and the average value is calculated.

### <Method for culturing cell, tissue or organ>

A method for culturing a cell, tissue or organ according to an aspect of the present invention comprises:
a step (A-a) of culturing a cell, tissue or organ using the culture vessel (α);
a step (B-a) of detaching the gas barrier layer (II-a) from the oxygen-permeable layer (I-a); and
a step (C-a) of further culturing the cell, tissue or organ using the culture vessel obtained in the step (B-a).

A method for culturing a cell, tissue or organ according to another aspect of the present invention comprises:
a step (A-b) of culturing a cell, tissue or organ using the culture vessel (β);
a step (B-b) of detaching the gas barrier layer (II-b) from the oxygen-permeable layer (I-b); and
a step (C-b) of further culturing the cell, tissue or organ using the culture vessel obtained in the step (B-b).

Step (A-a) or step (A-b) is a step of culturing a cell, tissue or organ using the culture vessel (α) or the culture vessel (β).

In the culture vessel (α) or the culture vessel (β), the water contact angle of the oxygen-permeable layer (I) is preferably 30 to 120°.

In the culture vessel (α) or the culture vessel (β), the oxygen-permeable layer (I) preferably contains a 4-methyl-1-pentene polymer (X).

In the culture vessel (α) or the culture vessel (β), the 4-methyl-1-pentene polymer (X) is preferably at least one polymer selected from a 4-methyl-1-pentene homopolymer (x1) and a copolymer (x2) of 4-methyl-1-pentene and at least one olefin selected from ethylene and an α-olefin having 3 to 20 carbon atoms (except for 4-methyl-1-pentene).

In the culture vessel (α) or the culture vessel (β), a culture surface, preferably a culture surface on a side where a contact with the culture medium and/or cells or the like is made, is preferably coated with a natural polymeric material, a synthetic polymeric material or an inorganic material.

In the culture vessel (α) or the culture vessel (β), the gas barrier layer (II) preferably contains polyethylene terephthalate (PET).

The method for culturing cells or the like in the culture medium is not limited, and may follow a known protocol, for which a commercially available culture medium or culture kit may be used.

The culture medium for use in the culture is not limited as long as it is a culture medium enabling cells or the like to proliferate, which may be appropriately selected according to a cell species used.

The amount of the culture medium used for the culture and the frequency of replacement of the culture medium is not limited. The culture temperature is not limited, but is typically about 25 to 40°C.

The culture period in step (A-a) or step (A-b) is not limited as long as cells or the like are cultured until sufficiently attaching to the culture vessel. The culture period is preferably 12 to 48 hours, more preferably 18 to 36 hours, and still more preferably 20 to 30 hours.

In step (A-a) or step (A-b), attachment is more easily improved because early culturing is performed under low oxygen supply conditions.

Step (B-a) or step (B-b) is a step of detaching the gas barrier layer (II) from the oxygen-permeable layer (I).

In the culture vessel (α), permeation of oxygen into the vessel is suppressed because the gas barrier layer (II) is present, but by detaching the gas barrier layer (II) from the oxygen-permeable layer (I), the oxygen-permeable layer is exposed, so that the culture vessel (α) or the culture vessel (β) allows oxygen to sufficiently permeate into the vessel.

The method for detaching the gas barrier layer (II) from the oxygen-permeable layer (I) is not limited, and an appropriate method may be used according to a method used for fixing the gas barrier layer (II) to the oxygen-permeable layer (I).

The time to carry out step (B-a) or step (B-b) is not limited, but is preferably a time after cells or the like sufficiently attach to the culture vessel. It is preferable to carry out step (B-a) or step (B-b) preferably 12 to 48 hours, more preferably 18 to 36 hours, and still more preferably 20 to 30 hours after cells or the like are seeded.

Step (C-a) or step (C-b) is a step of further culturing the cell, tissue or organ using the culture vessel obtained in the step (B-a) or step (B-b).

The method for culturing cells or the like in the culture medium is not limited, and may follow step (A-a) or step (A-b). The culture medium used in the culture, the amount of the culture medium used in the culture and the frequency of replacement of the culture medium may be identical to or different from those in step (A-a) or step (A-b) .

The culture period in step (C-a) or step (C-b) is not limited as long culturing is performed until cells or the like proliferate and reach a desired number of cells. The culture period is preferably 12 to 48 hours, more preferably 18 to 36 hours, and still more preferably 20 to 30 hours.

In step (C-a) or step (C-b), normal functions of cells or the like are easily maintained because cells or the like are cultured under sufficient oxygen supply conditions.

### Examples

Next, the present invention will be described in more detail by giving Examples, which should not be construed as limiting the present invention.

### (Methods)

### [Materials]

TPX film: a 4-methyl-1-pentene polymer film (thickness: 50 µm, manufactured by Mitsui Chemicals Tocello Inc., trade name: Opulent X-88B).

PET film 1: a commercially available PET film comprising a mixture of PET and a pressure sensitive adhesive (thickness: 80 µm, manufactured by BM Equipment Co., Ltd., trade name: qPCR plate seal (ABI, compression bonding)).

PET film 2: a commercially available PET film comprising a base material layer (PET layer, thickness: 75 µm) and a pressure sensitive adhesive layer (thickness: 10 pm) (NEION Film Coatings Corp., trade name: PET75-H270 (10)).

PP film: a commercially available PP film comprising a base material layer (PET layer, thickness: 60 µm) and a pressure sensitive adhesive layer (thickness: 10 pm) (NEION Film Coatings Corp., trade name: PP60-H270 (10)).

PDMS vessel: a commercial available 96-well culture vessel in which a bottom surface formed of a PDMS (dimethylpolysiloxane) film having a thickness of 350 µm (product name: G-plate, Model: V96WGPB-10 manufactured by VECELL Co., Ltd.).

PS vessel: a commercially available 24-well PS culture vessel in which a bottom surface made of PS (polystyrene) has a thickness of 1,000 µm (manufactured by Corning Incorporated).

### [Measurement of Physical Properties of Materials]

Using the TPX film, PET films 1 and 2, PP film, PDMS vessel and PS vessel as measurement sample, the oxygen permeability and the total light transmittance were measured by the methods described in detail below. Table 1 shows the results.

### [Measurement of Oxygen Permeability]

For the measurement samples, the oxygen permeation coefficient was measured in an environment at a temperature of 23°C and a humidity of 0% using a differential pressure type gas permeation rate measurement apparatus MT-C3 manufactured by Toyo Seiki Seisaku-sho, Ltd. The diameter of a measurement portion was 70 mm (the permeation area was 38.46 cm²). Since the oxygen permeation coefficient was thought to be high, the actual permeation area was set to 5.0 cm² by applying an aluminum mask to the sample in advance.

The value of the oxygen permeation coefficient [cm³·mm/(m²·24h·atm)] measured was divided by the thickness (µm) of the film (or the bottom surface of the culture vessel) to calculate the oxygen permeability [cm³/(m²·24h·atm)].

### [Measurement of Total light Transmittance]

For the measurement sample, the total light transmittance was measured according to JIS K 7361-1 using a hazemeter (NDH2000 manufactured by NIPPON DENSHOKU INDUSTRIES Co., Ltd. Co., Ltd.).

### [Measurement of Water Contact Angle]

The water contact angle was measured according to Japanese Industrial Standard JIS-R3257 (Testing method of wettability of glass substrate). A water droplet with a volume of 4 µL or less which can be considered to have a spherical shape under constant temperature and humidity conditions of 25 ± 5°C and 50 ± 10% was dropped to a surface of a measurement sample, and by a static drop method, the angle of a contact interface between the measurement sample and the water droplet within 1 minute immediately after the water droplet came into contact with the surface of the measurement sample was measured.

### [Preparation of Collagen Coating Solution]

A 0.1 M hydrochloric acid solution (for volumetric analysis, manufactured by FUJIFILM Wako Pure Chemical Corporation) was diluted 100-fold with water for injection (Japanese Pharmacopoeia, manufactured by Otsuka Pharmaceutical Co., Ltd.) to prepare a 0.001 M hydrochloric acid solution, which was sterilized by filtration. A solution of collagen at 3 mg/mL (Cellmatrix Typel-P, derived from pig tendon, manufactured by Nitta Gelatin Inc.) was diluted 6-fold with a 0.001 M hydrochloric acid solution to prepare a solution of collagen at 0.5 mg/mL.

### [Culture of Frozen Rat Primary Liver Cells]

A culture medium (A) was added to a centrifugal settling tube (50 mL) containing a cell suspension containing frozen rat primary liver cells (hepatocytes). The culture medium (A) was prepared by using 1.5 mL of fetal bovine serum (FBS, manufactured by FUJIFILM Wako Pure Chemical Corporation), 0.15 mL of a solution of L-proline (culture grade, manufactured by FUJIFILM Wako Pure Chemical Corporation) diluted to 3.0 g/mL with water for injection (manufactured by Fuso Pharmaceutical Industries, Ltd.), 1.5 µL of a solution of dexamethasone (biochemical grade, manufactured by FUJIFILM Wako Pure Chemical Corporation) diluted to 1 × 10⁻³ M with ethanol (molecular-biological grade, manufactured by FUJIFILM Wako Pure Chemical Corporation), 21 µL of a solution of hydrocortisone (culture grade, manufactured by FUJIFILM Wako Pure Chemical Corporation) diluted to 36 mM with ethanol, and a solution of BSA diluted to 1.0 mg/mL with water for injection, and adding 15 µL of a solution of an epidermal growth factor (EGF, for cell biological grade, manufactured by FUJIFILM Wako Pure Chemical Corporation) diluted to 20 µg/mL, 8.7 µL of an insulin solution (10 mg/mL in HEPESS, manufactured by Sigma-Aldrich Japan), 0.3 mL of a penicillin-streptomycin solution (containing penicillin at 5,000 units/mL and streptomycin at 5,000 µg/mL, culture grade, manufactured by FUJIFILM Wako Pure Chemical Corporation), and 13 mL of a D-MEM culture medium (containing D-glucose at 4,500 mg/mL, L-glutamine at 584 mg/mL, phenol red at 15 mg/mL, sodium pyruvate at 110 mg/mL and sodium hydrogen carbonate at 3,700 mg/mL, culture grade, manufactured by FUJIFILM Wako Pure Chemical Corporation). The cell density was adjusted by a method of adjusting the number of cells in a cell suspension containing frozen rat primary liver cells, and the cells were cultured at a cell density of 1.0 × 10⁵ cells/cm².

### [Measurement of Concentration of Oxygen in Culture Medium]

Twenty four hours after the cell seeding, the culture medium in the culture vessel was removed, 0.5 mL of the culture medium (A) was then freshly added, and the partial pressure (hPa) of oxygen in the culture medium was measured using a fluorescent oxygen sensor (FireSting Oxygen Monitor manufactured by BAS Inc.). The measurement was performed in a humidifying incubator, where a sensor was installed at a height of 80 um from the bottom surface of the culture vessel using a jack, followed by measurement for 1 hour. The partial pressure (hPa) of oxygen in the culture medium 1 hour after the start of the measurement was divided by 1,013 hPa (1 atm), and the obtained value was multiplied by 100. The calculated value (%) was defined as a concentration of oxygen in the culture medium. The measurement was performed in representative 3 wells, and the average value was calculated.

### [Measurement of Metabolic Activity Value]

The culture medium in the culture vessel was removed 24 hours or 48 hours after the cell seeding, luciferin-CEE diluted with a culture medium was then added, and the cells were further cultured for 3 hours. The cultured cells were transferred to a 96-well plate together with the culture medium containing luciferin-CEE, a mixed liquid of a luciferin detection reagent and a reconstitution buffer was then added, and the mixture was reacted at room temperature for 1 hour while being shielded from light. After an hour, the amount of luminescence (relative light unit, RLU) was measured by a luminometer.

For the protein amount, the solution of luciferin-CEE diluted with a culture medium was removed, 200 µL of PBS (-) was then added to the medium, and the cells were then collected in an Eppendorf tube using a cell scraper, and centrifuged (4°C, 22,000 xg, 10 min). Thereafter, the supernatant was removed, 100 µL of a 0.1 M sodium hydroxide solution was added, and the protein amount was then measured using Pierce^{™}BCA Protein Assay Kit (manufactured by Thermo Fisher Scientific, Inc.). The absorbance at a wavelength of 450 nm was measured by a plate reader (SPECTRA max PLUS384 manufactured by Molecular Devices, LLC).

The metabolic activity amount (pmol/L) of the luciferin-CEE solution obtained in the luminometer was measured using a P450-Glo^{™} CYP1A1 Assay kit (manufactured by Promega Corporation), and divided by the protein amount obtained from the absorbance and the reaction time of the luciferin-CEE solution, thereby calculating a metabolic activity value (pmol/min/mg protein). The measurement was performed in representative 3 wells, and the average value was calculated.

The metabolic activity maintenance ratio was calculated from the following equation. Metabolic activity maintenance ratio (%) = metabolic activity value 48 hours after seeding/metabolic activity value 24 hours after seeding × 100

### [Culture of Frozen Human Primary Liver Cells]

A culture medium (B) was added to a centrifugal settling tube (50 mL) containing a cell suspension containing frozen human primary liver cells (hepatocytes). The culture medium (B) was prepared by using 2.5 mL of fetal bovine serum (FBS, manufactured by FUJIFILM Wako Pure Chemical Corporation), 2.5 mL of antibiotic-antimycotic (containing penicillin at 10,000 units/mL, streptomycin at 10,000 ug/mL and amphotericin B at 25 ug/mL, Gibco^{™}), 0.05 mL of a solution of dexamethasone (biochemical grade, manufactured by FUJIFILM Wako Pure Chemical Corporation) diluted to 1 × 10⁻³ M with ethanol (molecular-biological grade, manufactured by FUJIFILM Wako Pure Chemical Corporation), 0.02 mL of an insulin solution (10 mg/mL in HEPESS, manufactured by Sigma-Aldrich Japan), 0.5 mL of a GlutaMAX solution (Gibco^{™}), 0.75 mL of a HEPES buffer solution (biochemical-grade buffering agent, manufactured by DOJINDO LABORATORIES.), 0.05 mL of L-Ascorbic Acid 2-Phosphate Trisodium Salt (manufactured by FUJIFILM Wako Pure Chemical Corporation) adjusted to 100 mM with water for injection, and a solution of BSA diluted to 1.0 mg/mL with water for injection, and adding 0.5 mL of a solution of an epidermal growth factor (EGF, cell biological grade, manufactured by FUJIFILM Wako Pure Chemical Corporation) diluted to 20 ug/mL, and 43.13 mL of William's E Medium (containing D-glucose at 2,000 mg/mL, phenol red at 10 mg/mL, sodium pyruvate at 25 mg/mL and sodium hydrogen carbonate at 2,200 mg/mL, culture grade, Giboco). The cell density was adjusted in the same manner as in the method of adjusting the number of cells in a cell suspension containing frozen rat primary liver cells, and the cells were cultured at a cell density of 1.0 × 10⁵ cells/cm².

Five hours after the cell seeding, the culture medium was replaced with a culture medium (C), and the cells were cultured for 19 hours. The culture medium (C) was prepared by using 2.5 mL of fetal bovine serum (FBS, manufactured by FUJIFILM Wako Pure Chemical Corporation), 1.25 mL of antibiotic-antimycotic (containing penicillin at 10,000 units/mL, streptomycin at 10,000 ug/mL and amphotericin B at 25 ug/mL, Gibco^{™}), 0.005 mL of a solution of dexamethasone (biochemical grade, manufactured by FUJIFILM Wako Pure Chemical Corporation) diluted to 1 × 10⁻³ M with ethanol (molecular-biological grade, manufactured by FUJIFILM Wako Pure Chemical Corporation), 0.5 mL of a GlutaMAX solution (Gibco^{™}), 0.75 mL of a HEPES buffer solution (biochemical-grade buffering agent, manufactured by DOJINDO LABORATORIES.), 0.5 mL of ITS premix (manufactured by Corning Incorporated), 0.05 mL of L-Ascorbic Acid 2-Phosphate Trisodium Salt (manufactured by FUJIFILM Wako Pure Chemical Corporation) adjusted to 100 mM with water for injection, and a solution of BSA diluted to 1.0 mg/mL with water for injection, and adding 0.5 mL of a solution of an epidermal growth factor (EGF, cell biological grade, manufactured by FUJIFILM Wako Pure Chemical Corporation) diluted to 20 ug/mL, 1.0 mL of HepExtended Supplement (Gibco^{™}), and 42.945 mL of William's E Medium (containing D-glucose at 2,000 mg/mL, phenol red at 10 mg/mL, sodium pyruvate at 25 mg/mL and sodium hydrogen carbonate at 2,200 mg/mL, culture grade, Gibco^{™}) .

### [Evaluation of Cell Attachment]

To a culture vessel, 0.5 mL of a cell suspension of frozen human primary liver cells was added to seed the cells at a cell density of 1 × 10⁵ cells/cm². The cells were incubated at 37°C under 5% CO₂, a state after culturing for 24 hours was observed under a microscope, and evaluated according to the following criteria. A picture was taken under the microscope.

AA: A state is observed in which liver cells attach to and spread over the culture surface.

BB: A state is observed in which liver cells attach to the culture surface, but curl and do not spread, or partially peel.

### [Evaluation of Peelability]

A film (any of PET films 1 and 2 and a PP film) adhered to a film forming the bottom surface of the culture vessel was peeled from an edge portion of the film. Occurrence or non-occurrence of breakage of the film forming the bottom surface of the culture vessel and the peeled film (any of PET films 1 and 2 and a PP film), and the like were confirmed, followed by evaluation based on the following criteria.

AA: The film forming the bottom surface of the culture vessel and the peeled film (any of PET films 1 and 2 and a PP film) did not break, and warpage of the film forming the bottom surface of the culture vessel was not observed, which indicates good peelability.

BB: The film forming the bottom surface of the culture vessel and/or the peeled film (any of PET films 1 and 2 and a PP film) broke, or the film forming the bottom surface of the culture vessel warped, which indicates poor peelability.

### [Evaluation of Peeling Strength]

Release paper on a film cut into 25 mm × 150 mm (any of PET films 1 and 2 and a PP film) was peeled, the peeled surface was attached to the upper surface of a TPX film or a PDMS film cut into 50 mm × 150 mm, and a 2 kg roller was made to reciprocate once. This was held in an environment at 23°C and RH 50% for 30 minutes, and the peeling strength [N/25 mm] was then measured under conditions of a peeling angle of 180° and a pulling rate of 10 mm/min in an environment at 23°C and RH 50% according to JIS Z 0237 using a peeling tester (Strograph E-S manufactured by Toyo Seiki Seisaku-sho, Ltd.). The test was conducted three times, and the average value was calculated.

### [Example 1]

In a chamber filled with a nitrogen flow, plasma treatment (treatment rate: 2 m/min, power 4.5 kW, two reciprocations) was performed on a TPX film using a normal-pressure plasma surface treatment apparatus (manufactured by Sekisui Chemical Company, Limited).

The plasma-treated TPX film was cut into a size of 8 cm × 12 cm, and brought into close contact with a frame of a bottomless well-type 24-well vessel made of PS, with a medical-grade pressure sensitive adhesive (manufactured by 3M Company) interposed therebetween, thereby producing a 24-well culture plate in which a TPX film forms the bottom surface of the culture plate.

To the TPX film at the bottom surface of the culture plate, the PET film 1 was compression-bonded at a certain pressure on a side opposite to the vessel frame by making a pressing rubber roller (roller width: 45 mm, roller mass: 2 kg) reciprocate twice. In this way, a 24-well culture plate was produced in which a laminate of the TPX film and the PET film 1 forms the bottom surface of the culture plate. That is, a surface of the PET film 1, from which release paper had been peeled, was adhered to the lower surface of the TPX film forming the bottom surface of the culture plate.

Thereafter, the 24-well culture plate was packaged in a gamma ray-resistant bag, and sterilized by irradiation with a gamma ray of 10 kGy.

To each well of the 24-well culture plate produced as described above, 0.5 mL of a collagen solution at 0.5 mg/mL was added, and an excess collagen solution was then removed. This was allowed to stand at room temperature for 30 to 60 minutes, then washed with Dulbecco PBS (-), and dried overnight at room temperature. The five PET film 1-attached 24-well culture plates coated with collagen (each also called a culture vessel (1)) were prepared. They were culture vessels (1) A, B, C, D and E, respectively. Thereafter, the water contact angle of the culture vessel (1) was measured. Table 1 shows the results.

To the culture vessels (1) A to D, among the culture vessels (1) A to E produced as described above, a culture medium (0.5 mL) containing frozen rat primary liver cells was added with a micropipette to seed the cells at a cell density of 1.0 × 10⁵ cells/cm². Culturing was started at 37°C under 5% CO₂.

Twenty four hours after the cell seeding, the culture vessels A to D were taken out from the incubator.

The culture vessels (1) A and B were returned to the incubator after the PET film at the bottom surface was peeled, and the cells were further cultured for 24 hours.

In the culture vessel (1) C, the concentration of dissolved oxygen in the culture medium was measured.

In the culture vessel (1) D, a metabolic activity value was measured as an index of a normal function of liver cells.

Forty eight hours after the cell seeding, the culture vessel (1) A, from which the PET film 1 had been peeled, was taken out from the incubator, and the concentration of dissolved oxygen was measured.

Forty eight hours after the cell seeding, the culture vessel (1) B, from which the PET film 1 had been peeled, was taken out from the incubator, and the metabolic activity value was measured.

Table 1 shows the results.

To the culture vessel (1) E, among the culture vessels (1) produced as described above, a culture medium (0.5 mL) containing frozen human primary liver cells was added with a micropipette to seed the cells at a cell density of 1.0 × 10⁵ cells/cm². Culturing was started at 37°C under 5% CO₂.

Twenty four hours after the cell seeding, the culture vessel (1) E was taken out from the incubator, the PET film 1 at the bottom surface was peeled, and peelability and cell attachment were evaluated. The results are shown in Table 1 and Figure 2.

### [Example 2]

In Example 2, frozen rat liver cells and frozen human liver cells were cultured and evaluated in the same manner as in Example 1 except that the PET film 1 at the bottom surface of the culture vessel (1) was changed to the PP film, and the surface treatment of the TPX film was changed to corona treatment using a table-type corona treatment apparatus (manufactured by Kasuga electric works Ltd.) (treatment rate: 3 m/min, power: 0.5 kW, two reciprocations). Table 1 shows the results.

### [Example 3]

In Example 3, frozen rat liver cells and frozen human liver cells were cultured and evaluated in the same manner as in Example 1 except that the PET film 1 at the bottom surface of the culture vessel (1) was changed to the PET film 2. Table 1 shows the results.

### [Example 4]

To the PDMS film forming the bottom surface of a PDMS vessel, the PET film 2 was compression-bonded at a certain pressure by making a pressing rubber roller (roller width: 45 mm, roller mass: 2 kg) reciprocate twice. In this way, a 96-well culture plate was produced in which a laminate of the PDMS film and the PET film 2 forms the bottom surface of the culture plate. To each well, 0.1 mL of a collagen solution at 0.5 mg/mL was added, and an excess collagen solution was then removed. This was allowed to stand at room temperature for 30 to 60 minutes, then washed with Dulbecco PBS (-), and dried overnight at room temperature. In Example 4, frozen rat liver cells and frozen human liver cells were cultured and evaluated in the same manner as in Example 1. Table 1 shows the results.

### [Reference Example 1]

In Reference Example 1, frozen rat liver cells were cultured and evaluated in the same manner as in Example 1 except that the PET film 1 at the bottom surface of the culture vessel (1) was not peeled. Table 2 shows the results. Frozen human liver cells were not either cultured or evaluated.

### [Comparative Example 1]

In Comparative Example 1, frozen rat liver cells were cultured and evaluated in the same manner as in Example 1 except that a commercially available 24-well PS culture vessel (PS) was used instead of the 24-well culture plate in which a laminate of the TPX film and the PET film 1 forms the bottom surface of the culture plate. Table 2 shows the results. Frozen human liver cells were not either cultured or evaluated.

### [Comparative Example 2]

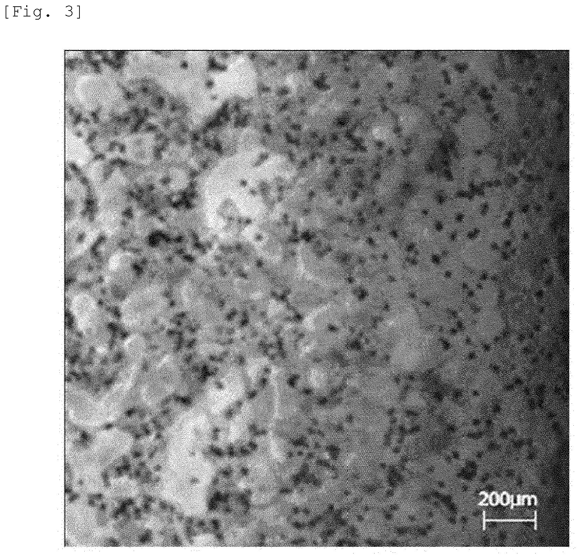

In Comparative Example 2, frozen rat liver cells and frozen human liver cells were cultured and evaluated in the same manner as in Example 1 except that the 24-well culture plate in which the TPX film forms the bottom surface of the culture plate was used, and the PET film 1 was not attached. The results are shown in Table 2 and Figure 3.

**[Table 1]**

| | | | | Example 1 | Example 2 | Example 3 | Example 4 |
|---|---|---|---|---|---|---|---|
| Culture vessel | Upper layer | Material | | TPX | TPX | TPX | PDMS |
| | | Thickness | µm | 50 | 50 | 50 | 350 |
| | | Oxygen permeation coefficient | (cm³ × mm)/(m² × 24 h × atm) | 1,912 | 1,912 | 1,912 | 19,121 |
| | | Oxygen permeability | cm³/(m² × 24 h × atm) | 38,240 | 38,240 | 38,240 | 54,631 |
| | | Total light transmittance | % | 93.7 | 93.7 | 93.7 | 80.1 |
| | | Collagen coating | | Present | Present | Present | Present |
| | | Water contact angle | ○ | 51.3 | 72.8 | 51.3 | 62.0 |
| | Lower layer (peelable layer) | Material | | PET1 | PP | PET2 | PET2 |
| | | Thickness of base material | µm | 80 | 60 | 75 | 75 |
| | | Thickness of pressure sensitive adhesive layer | µm | - | 10 | 10 | 10 |
| | | Oxygen permeation coefficient | (cm³ × mm)/(m² × 24 h × atm) | 1.6 | 105 | 1.0 | 1.0 |
| | | Oxygen permeability | cm³/(m² × 24 h × atm) | 20 | 1500 | 12 | 12 |
| | | Total light transmittance | % | 92.1 | 90.3 | 92.1 | 92.1 |
| | | Peelability | | AA | AA | AA | AA |
| | | Peeling strength | N/25 mm | 0.11 | 0.10 | 0.03 | 0.03 |
| Frozen rat liver cells | 24 Hours after cell seeding | Peelable layer | | Present | Present | Present | Present |
| | | Concentration of oxygen in culture medium | % | 2.1 | 2.1 | 2.1 | 2.3 |
| | | Metabolic activity value | pmol/min/mg protein | 0.74 | 0.70 | 0.74 | 0.68 |
| | 48 Hours after cell seeding | Peel layer | | None | None | None | None |
| | | Concentration of oxygen in culture medium | % | 14 | 13.8 | 14 | 14.8 |
| | | Metabolic activity value | pmol/min/mg protein | 0.51 | 0.49 | 0.51 | 0.45 |
| | Metabolic activity maintenance ratio | | % | 68.9 | 70.0 | 68.9 | 66.2 |
| Frozen human liver cells | 24 Hours after cell seeding | Cell attachment evaluation | | AA | AA | AA | AA |

**[Table 2]**

| | | | | Reference Example 1 | Comparative Example 1 | Comparative Example 2 |
|---|---|---|---|---|---|---|
| Culture vessel | Upper layer | Material | | TPX | PS | TPX |
| | | Thickness | µm | 50 | 1,000 | 50 |
| | | Oxygen permeation coefficient | (cm³ × mm)/(m² × 24 h × atm) | 1,912 | 203 | 1,912 |
| | | Oxygen permeability | cm³/(m² × 24 h × atm) | 38,240 | 203 | 38,240 |
| | | Total light transmittance | % | 93.7 | 89.1 | 93.7 |
| | | Collagen coating | | Present | Present | Present |
| | | Water contact angle | ○ | 51.3 | 60.3 | 51.3 |
| | Lower layer (peelable layer) | Material | | PET1 | - | - |
| | | Thickness of base material | µm | 80 | - | - |
| | | Thickness of pressure sensitive adhesive layer | µm | - | | |
| | | Oxygen permeation coefficient | (cm³ × mm)/(m² × 24 h × atm) | 1.6 | - | - |
| | | Oxygen permeability | cm³/(m² × 24 h × atm) | 20 | - | - |
| | | Total light transmittance | % | 92.1 | - | - |
| | | Peelability | | - | - | - |
| | | Peeling strength | N/25 mm | 0.11 | - | - |
| Frozen rat liver cells | 24 Hours after cell seeding | Peelable layer | | Present | None | None |
| | | Concentration of oxygen in culture medium | % | 2.3 | 0.2 | 14.3 |
| | | Metabolic activity value | pmol/min/mg protein | 0.75 | 0.70 | 1.15 |
| | 48 Hours after cell seeding | Peelable layer | | Present | None | None |
| | | Concentration of oxygen in culture medium | % | - | - | - |
| | | Metabolic activity value | pmol/min/mg protein | 0.38 | 0.33 | 0.72 |
| | Metabolic activity maintenance ratio | | % | 50.7 | 47.1 | 62.6 |
| Frozen human liver cells | 24 Hours after cell seeding | Cell attachment evaluation | | - | - | BB |

### (Results)

In all Examples 1 to 4, good cell attachment 24 hours after cell seeding was presented. In addition, a good metabolic activity maintenance ratio was presented, and it was possible to suppress a decrease in metabolic activity of cells which may occur until 48 hours after cell seeding from 24 hours after cell seeding. It was revealed that the culture vessels of Examples 1 to 4 allowed liver cells to favorably attach, and enabled maintenance of normal functions of liver cells.

In Reference Example 1, a low metabolic activity maintenance ratio was presented, and the metabolic activity of cells decreased until 48 hours after cell seeding from 24 hours after cell seeding. This may be because a PET film with a low oxygen permeability was not peeled during culture, and remained attached, and oxygen was not sufficiently supplied to cells until 48 hours after cell seeding from 24 hours after cell seeding.

In Comparative Example 1, a low metabolic activity maintenance ratio was presented, and the metabolic activity of cells decreased until 48 hours after cell seeding from 24 hours after cell seeding. This may be because PS with a low oxygen permeability formed the bottom surface of the culture vessel, and oxygen was not sufficiently supplied to cells until 48 hours after cell seeding from 24 hours after cell seeding.

In Comparative Example 2, a good metabolic activity maintenance ratio was presented, and it was possible to suppress a decrease in metabolic activity of cells which may occur until 48 hours after cell seeding from 24 hours after cell seeding. However, 24 hours after cell seeding, cells did not attach to the culture vessel, and remained separate. This may be because only a TPX film with a high oxygen permeability formed the bottom surface of the culture vessel, and oxygen was excessively supplied to cells until 24 hours after cell seeding from the time immediately after cell seeding.

### <Incorporation by reference>

This application claims priority based on JP2021-182633A filed to Japanese Patent Office on November 9, 2021, the disclosure of which is incorporated herein in its entirety.

## Claims

1. A culture vessel for culturing a cell, tissue or organ,
wherein the culture vessel comprises a bottom surface component and a lateral wall component,
the bottom surface component and the lateral wall component are bonded to each other to form at least one culture space,
at least a part of the bottom surface component includes at least an oxygen-permeable layer (I-b) and a gas barrier layer (II-b),
the gas barrier layer (II-b) is stacked under the oxygen-permeable layer (I-b) so as to be detachable from the oxygen-permeable layer (I-b),
the gas barrier layer (II-b) satisfies the following requirement (5), and
the oxygen-permeable layer (I-b) satisfies the following requirement (6):
(5) an oxygen permeability P(II-b) at a temperature of 23°C and a humidity of 0% is 3,000 cm³/(m²·24h·atm) or less; and
(6) an oxygen permeability P(I-b) at a temperature of 23°C and a humidity of 0% is equal to or more than 6.0 times the P(II-b) .

2. The culture vessel according to claim 1, wherein the gas barrier layer (II-b) contains at least one selected from the group consisting of polyethylene terephthalate (PET) and polyolefin, and satisfies the following requirement (7) or (8) :
(7) containing a mixture of at least one selected from the group consisting of polyethylene terephthalate (PET) and polyolefin and a pressure sensitive adhesive; or
(8) including a layer containing at least one selected from the group consisting of polyethylene terephthalate (PET) and polyolefin, and a layer containing a pressure sensitive adhesive.

3. The culture vessel according to claim 1 or 2, wherein a peeling strength of the gas barrier layer (II-b) against the oxygen-permeable layer (I-b) is 0.01 to 0.20 N/25 mm as measured by the following method:
[a test is conducted in which the gas barrier layer (II-b) having a width of 25 mm is attached to the oxygen-permeable layer (I-b), and the gas barrier layer (II-b) is then peeled from the oxygen-permeable layer (I-b) at a rate of 10 mm/min at a peeling angle of 180° using a peeling tester].

4. The culture vessel according to claim 1 or 2, wherein the oxygen-permeable layer (I-b) contains a 4-methyl-1-pentene polymer (X).

5. The culture vessel according to claim 4, wherein the 4-methyl-1-pentene polymer (X) is at least one polymer selected from a 4-methyl-1-pentene homopolymer (x1) and a copolymer (x2) of 4-methyl-1-pentene and at least one olefin selected from ethylene and an α-olefin having 3 to 20 carbon atoms (except for 4-methyl-1-pentene).

6. The culture vessel according to claim 1 or 2, wherein the P(I-b) is 20,000 to 60,000 cm³/(m²·24h·atm).

7. The culture vessel according to claim 1 or 2, wherein a total light transmittance of a region of the bottom surface component which includes the oxygen-permeable layer (I-b) and the gas barrier layer (II-b) is 70% or more as measured according to JIS K 7361-1.

8. The culture vessel according to claim 1 or 2, wherein a total light transmittance of the oxygen-permeable layer (I-b) is 70% or more as measured according to JIS K 7361-1.

9. The culture vessel according to claim 1 or 2, wherein a total light transmittance of the gas barrier layer (II-b) is 70% or more as measured according to JIS K 7361-1.

10. The culture vessel according to claim 1 or 2, wherein a water contact angle of the oxygen-permeable layer (I-b) is 30 to 120°.

11. The culture vessel according to claim 1 or 2, wherein the culture surface of the bottom surface component is coated with a natural polymeric material, a synthetic polymeric material or an inorganic material.

12. A method for culturing cell, tissue or organ, comprising:
a step (A-b) of culturing a cell, tissue or organ using the culture vessel according to claim 1 or 2;
a step (B-b) of detaching the gas barrier layer (II-b) from the oxygen-permeable layer (I-b); and
a step (C-b) of further culturing the cell, tissue or organ using the culture vessel obtained in the step (B-b).

13. A method for producing the culture vessel according to claim 1 or 2,
the method comprising
a step of attaching the bottom surface component to the lateral wall component.
